(19) Europäisches Patentamt European Patent Office Office européen des brevets

(11) EP 3 240 904 B1

(12) FASCICULE DE BREVET EUROPEEN

(45) Date de publication et mention de la délivrance du brevet:
22.04.2020 Bulletin 2020/17

(51) Int Cl.:
C12P 19/26 (2006.01)  C08B 37/08 (2006.01)
C12P 21/06 (2006.01)  C07K 14/435 (2006.01)

(21) Numéro de dépôt: 15830826.2

(22) Date de dépôt: 30.12.2015

(86) Numéro de dépôt international:
PCT/FR2015/053783

(87) Numéro de publication internationale:
WO 2016/108035 (07.07.2016 Gazette 2016/27)

(54) **PRODUCTION DE CHITINE ET/OU DE CHITOSAN À PARTIR D'INSECTES PAR HYDROLYSE ENZYMATIQUE COMPORTANT UNE COMBINAISON D'ÉTAPES PRÉALABLES**

HERSTELLUNG VON CHITIN UND/ODER CHITOSAN AUS INSEKTEN DURCH ENZYMATISCHE HYDROLYSE MIT EINER KOMBINATION AUS VOR DER ENZYMATISCHEN HYDROLYSE DURCHGEFÜHRTEN SCHRITTEN

PRODUCTION OF CHITIN AND/OR CHITOSAN FROM INSECTS BY MEANS OF ENZYMATIC HYDROLYSIS, COMPRISING A COMBINATION OF STEPS PERFORMED PRIOR TO THE ENZYMATIC HYDROLYSIS

(84) Etats contractants désignés:
AL AT BE BG CH CY CZ DE DK EE ES FI FR GB GR HR HU IE IS IT LI LT LU LV MC MK MT NL NO PL PT RO RS SE SI SK SM TR

(30) Priorité: 31.12.2014 FR 1463513

(43) Date de publication de la demande:
08.11.2017 Bulletin 2017/45

(73) Titulaire: Ynsect
91058 Évry-Courcouronnes Cedex (FR)

(72) Inventeurs:
• BEREZINA, Nathalie
  75005 Paris (FR)
• HUBERT, Antoine
  94140 Alfortville (FR)
• BERRO, Fabrice
  75001 Paris (FR)
• LEVON, Jean-Gabriel
  75011 Paris (FR)
• LE ROUX, Karine
  91490 Milly La Foret (FR)
• SOCOLSKY, Cecilia
  59800 Lille (FR)
• SANCHEZ, Lorena
  91260 Juvisy-sur-Orge (FR)
• LAURENT, Sophie
  44200 Nantes (FR)

(74) Mandataire: Santarelli
49, avenue des Champs-Elysées
75008 Paris (FR)

(56) Documents cités:
WO-A1-2012/168618    CN-A- 1 297 691
CN-A- 101 775 085    CN-B- 101 144 097
FR-A1- 2 927 336     US-A- 4 958 011

• DATABASE WPI Week 201301 2012 Thomson Scientific, London, GB; AN 2012-M24188 XP002747930, -& CN 102 558 387 A (QINGDAO ZHONGREN PHARMACY CO LTD) 11 juillet 2012 (2012-07-11)
• Nitar Nwe ET AL: "Chitin and Chitosan from Terrestrial Organisms" In: "Chitin, Chitosan, Oligosaccharides and Their Derivatives: Biological Activities and Applications", 1 octobre 2010 (2010-10-01), CRC Press, XP055221223, ISBN: 978-1-4398-1603-5 pages 3-10, alinéa [1.2.2]
• MARÍA CECILIA GORTARI ET AL: "Biotechnological processes for chitin recovery out of crustacean waste: A mini-review", EJB ELECTRONIC JOURNAL OF BIOTECHNOLOGY, vol. 16, no. 3, 15 mai 2013 (2013-05-15), pages 1-18, XP055221276, CL ISSN: 0717-3458, DOI: 10.2225/vol16-issue3-fulltext-10

- Wolfram M Brück ET AL: "Chitin and Chitosan from Marine Organisms" In: "Chitin, Chitosan, Oligosaccharides and Their Derivatives: Biological Activities and Applications", 1 octobre 2010 (2010-10-01), CRC Press, XP055221232, ISBN: 978-1-4398-1603-5 pages 11-23, pages 14-19, alinéa 2.3-2.3.3; figures 2.3, 2.4
- LAILA MANNI ET AL: "An Oxidant- and Solvent-Stable Protease Produced by Bacillus cereus SV1: Application in the Deproteinization of Shrimp Wastes and as a Laundry Detergent Additive", APPLIED BIOCHEMISTRY AND BIOTECHNOLOGY, vol. 160, no. 8, 1 avril 2010 (2010-04-01) , pages 2308-2321, XP055221571, United States ISSN: 0273-2289, DOI: 10.1007/s12010-009-8703-z
- JUAN WANG ET AL: "Housefly larvae hydrolysate: orthogonal optimization of hydrolysis, antioxidant activity, amino acid composition and functional properties", BMC RESEARCH NOTES, BIOMED CENTRAL LTD, GB, vol. 6, no. 1, 17 mai 2013 (2013-05-17), page 197, XP021151554, ISSN: 1756-0500, DOI: 10.1186/1756-0500-6-197
- DAI CHUNHUA ET AL: "Angiotensin I-converting enzyme (ACE) inhibitory peptide derived fromTenebrio molitor(L.) larva protein hydrolysate", EUROPEAN FOOD RESEARCH AND TECHNOLOGY, SPRINGER VERLAG, HEIDELBERG, DE, vol. 236, no. 4, 1 février 2013 (2013-02-01), pages 681-689, XP035311054, ISSN: 1438-2377, DOI: 10.1007/S00217-013-1923-Z [extrait le 2013-02-01]

**Description**

La présente invention concerne un procédé de préparation de chitine et/ou de chitosan à partir d'insectes. Plus particulièrement, l'invention concerne un procédé de préparation de chitine et/ou de chitosan par hydrolyse enzymatique de cuticules d'insectes.

Selon l'invention, par « chitine », on entend tout type de dérivé chitinique, c'est-à-dire de dérivé de polysaccharides comportant des unités N-acétyl-glucosamine et des unités D-glucosamines, en particulier les copolymères chitine-polypeptides (parfois désignés sous l'appellation « composite chitine-polypeptides »).

La chitine serait le deuxième polymère le plus synthétisé dans le monde vivant après la cellulose. En effet, la chitine est synthétisée par de nombreuses espèces du monde vivant: elle constitue en partie l'exosquelette des crustacés et des insectes et la paroi latérale qui entoure et protège les champignons. Plus particulièrement, chez les insectes, la chitine constitue ainsi 3 à 60% de leur exosquelette.

Par « chitosan », on entend selon la présente invention les produits de désacétylation de la chitine. La limite usuelle entre le chitosan et la chitine est déterminée par le degré d'acétylation : un composé ayant un degré d'acétylation inférieur à 50% est nommé chitosan, au-delà, un composé ayant un degré d'acétylation supérieur à 50% est nommé chitine.

Les applications de la chitine et/ou du chitosan sont nombreuses: cosmétique (composition cosmétique), médicale et pharmaceutique (composition pharmaceutique, traitement des brûlures, biomatériaux, pansements cornéens, fils chirurgicaux), diététique et alimentaire, technique (agent filtrant, texturant, floculant ou adsorbant notamment pour la filtration et dépollution de l'eau), etc. En effet, la chitine et /ou le chitosan sont des matériaux biocompatibles, biodégradables et non toxiques.

L'extraction traditionnelle de la chitine s'effectue par voie chimique à partir de crustacés, de céphalopodes, mais aussi, de manière plus exceptionnelle, de champignons. Cette voie emploie de grandes quantités de réactifs (tel que l'acide chlorhydrique, l'hydroxyde de sodium et des agents de blanchiment) qui ont pour effet de dénaturer la structure de la chitine telle qu'elle existe à l'état naturel, par exemple telle que présente dans la carapace des crustacés. En outre, la plupart des réactifs chimiques sont nocifs pour l'homme et l'environnement et génèrent d'importants volumes d'effluents à traiter. Enfin, la chitine et/ou le chitosan issu(s) de crustacés peuvent générer des réactions allergiques chez les personnes sensibles.

Une autre voie d'extraction de la chitine est la voie enzymatique. Cette voie est considérée comme plus douce, permettant ainsi de mieux préserver la chitine et/ou le chitosan. Toutefois, la chitine obtenue par cette voie est d'une couleur brunâtre, nécessitant des étapes de purification afin d'obtenir une poudre valorisable, c'est-à-dire de couleur blanche. Les procédés existants comportent donc généralement une ou plusieurs étape(s) visant à débarrasser la chitine de ses impuretés, telle qu'une étape de déminéralisation à l'acide réalisée préalablement à l'hydrolyse enzymatique et/ou une étape de blanchiment de la chitine avec un agent oxydant, réalisée de manière subséquente à l'hydrolyse enzymatique. Ces deux étapes de purification de la chitine ont malheureusement pour effet d'altérer la structure chimique de la chitine.

Le travail des inventeurs a permis de mettre en évidence qu'il était possible d'obtenir une chitine à la fois plus pure et de structure plus proche de la structure originelle de la chitine en effectuant une combinaison spécifique d'étapes préalables à l'hydrolyse, à savoir une étape de pressage et une étape de traitement des cuticules d'insectes par un agent oxydant.

L'invention concerne donc un procédé de production de chitine et/ou de chitosan à partir de cuticules d'insectes, comportant les étapes suivantes :

(i) le pressage des cuticules d'insectes, puis,

(ii) l'hydrolyse enzymatique des cuticules d'insectes par une enzyme protéolytique,

un traitement des cuticules avec un agent oxydant ayant été effectué préalablement à l'hydrolyse enzymatique.

Par « insectes », on entend des insectes à n'importe quel stade de développement, tel qu'un stade adulte, larvaire ou un stade de nymphe. De préférence, les insectes mis en œuvre dans le procédé selon l'invention sont comestibles.

Plus particulièrement, les insectes peuvent être choisis parmi le groupe constitué par les coléoptères, les diptères, les lépidoptères, les isoptères, les orthoptères, les hyménoptères, les blattoptères, les hémyptères, les hétéroptères, les éphéméroptères et les mécoptères, de préférence, parmi les coléoptères, les diptères, les orthoptères et les lépidoptères.

Préférentiellement, les insectes sont choisis parmi le groupe constitué par *Tenebrio molitor, Hermetia illucens, Galleria mellonella, Alphitobius diaperinus, Zophobas morio, Blattera fusca, Tribolium castaneum, Rhynchophorus ferrugineus, Musca domestica, Chrysomya megacephala, Locusta migratoria, Schistocerca gregaria, Acheta domesticus* et *Samia ricini.*

Plus préférentiellement, les insectes sont choisis parmi le groupe constitué par *Tenebrio molitor, Hermetia illucens, Galleria mellonella, Alphitobius diaperinus, Zophobas morio, Blattera fusca, Musca domestica, Chrysomya megacephala, Locusta migratoria, Schistocerca gregaria, Acheta domesticus* et *Samia ricini,* et plus préférentiellement encore, *T. molitor.*

Une ou plusieurs espèces d'insectes peuvent être utilisées dans le procédé selon l'invention, de préférence une seule espèce d'insecte. Si plusieurs espèces sont utilisées, on choisira avantageusement deux espèces proches telles que par exemple, *Hermetia illucens* et *Musca domestica.*

Les insectes sont de préférence élevés et non prélevés dans la nature. Par exemple, les insectes sont élevés dans une ferme d'insectes. L'élevage des insectes dans une ferme spécifique permet non seulement de contrôler et d'éliminer les risques associés aux maladies véhiculées par des insectes, mais également de limiter les risques associés à la toxicité des produits alimentaires dérivés des insectes due par exemple à la présence d'insecticides. En outre, l'élevage permet de contrôler la qualité de l'approvisionnement en insectes et de limiter les coûts d'approvisionnement.

Par « cuticules d'insectes », on vise non seulement les cuticules une fois celles-ci séparées des insectes, mais également les cuticules incluant tout ou partie des autres constituants de l'insecte, y compris l'insecte dans son intégralité. En effet, il est possible d'appliquer le procédé selon l'invention à l'insecte complet, tels que des insectes broyés, ou bien à seulement une partie des insectes comportant les cuticules, par exemple les exuvies et/ou les mues d'insectes, séparées naturellement et collectées par un procédé adéquat.

La cuticule est la couche externe (ou exosquelette) sécrétée par l'épiderme des insectes. Elle est en général formée de trois couches :

- l'épicuticule, qui est la couche la plus fine et la plus externe de la cuticule (inférieure à 4 $\mu$m) ; cette couche est imperméable à l'eau et comporte une couche de cire imperméabilisante, ainsi que des protéines et de la chitine, en quantité moindre ;
- l'exocuticule, qui est la couche intermédiaire de la cuticule ; elle est composée essentiellement de protéines durcies, les tannées, qui sont responsables de la rigidité de la cuticule, de chitine et éventuellement de mélanine ; et
- l'endocuticule, qui est une couche fine, flexible, constituée d'un mélange de protéines et de chitine.

Le pressage des cuticules d'insectes a pour objectif principal d'éliminer un jus de presse riche en matière grasse et/ou d'enrichir le gâteau de presse en substrat pour l'hydrolyse.

Dans le procédé selon l'invention, le pressage des cuticules d'insectes permet d'obtenir un gâteau de presse comportant une teneur en huile (ou lipides) inférieure ou égale à 20%, préférentiellement, inférieure ou égale à 15%, plus préférentiellement, inférieure ou égale à 12%, encore plus préférentiellement, inférieure ou égale à 10%.

Dans la présente demande, les gammes de valeurs s'entendent bornes incluses. De même, lorsqu' « environ » ou « de l'ordre de » précède un nombre, cela équivaut à plus ou moins 10% de la valeur de ce nombre.

Par ailleurs, afin d'enrichir le gâteau de presse en substrat pour l'hydrolyse, le pressage des cuticules d'insectes permet d'obtenir un gâteau de presse présentant une teneur en matière sèche comprise entre 30% et 60%, préférentiellement, comprise entre 40% et 55%, et plus préférentiellement comprise entre 45% et 50%.

Tout système de presse peut être utilisé pour réaliser le pressage des cuticules d'insectes, tel que par exemple, une presse mono-vis ou bi-vis (presse bi-vis de type Angel), un filtre-presse (filtre-presse de type Choquenet), une presse à plateaux, etc. Ces systèmes sont bien connus de l'homme du métier qui est à même de déterminer les conditions de pressage afin d'obtenir les teneurs en huile et/ou en eau mentionnées ci-avant.

Dans le procédé selon l'invention, le pressage des cuticules d'insectes est suivi d'une hydrolyse enzymatique.

De préférence, l'hydrolyse enzymatique est effectuée par au moins une enzyme protéolytique, de préférence une protéase. Dans la présente demande, les noms ou suffixes « peptidase » et « protéase » sont utilisés indifféremment pour désigner une enzyme lysant une liaison peptidique des protéines. Avantageusement, celle-ci est effectuée pendant une durée de 4 à 8h, préférentiellement, pendant 4 à 5h, à une température de 40 à 60 °C, préférentiellement, 45 à 55 °C et à un pH compris entre 6 et 8, préférentiellement entre 6,5 et 7,5.

L'hydrolyse enzymatique peut être réalisée avec une seule protéase ou alternativement avec un mélange d'enzymes contenant au moins une protéase, plus préférentiellement un mélange d'enzymes contenant plusieurs protéases, tel qu'un mélange contenant une endoprotéase et une exoprotéase, ou une protéase et une polysaccharase.

De préférence, la protéase est choisie parmi le groupe constitué par les aminopepdidases, les métallocarboxypeptidases, les endopeptidases sérine, les endopeptidases cystéine, les endopeptidases aspartiques, les métalloendopeptidases.

Avantageusement, les enzymes peuvent être choisies parmi les suivantes :

| Enzyme(s) | Classe | Numéro EC | Fournisseur | Ville | Pays |
|---|---|---|---|---|---|
| Flavourzyme | Aminopeptidases | EC 3.4.11.1 | Novozyme | Bagsvaerd | Danemark |
| Fungal protease 500 | | EC 3.4.11.1 | Bio-Cat | Troy | Etats-Unis |
| Kojizyme | | EC 3.4.11.1 | Novozyme | Bagsvaerd | Danemark |
| Protex P | Endopeptidases sérine | EC 3.4.21 | Genencor International B.V. | Leiden | Pays-Bas |
| Chymotrypsine | | EC 3.4.21.1 | Novozyme | Bagsvaerd | Danemark |
| Protamex | | EC 3.4.21 | Novozyme | Bagsvaerd | Danemark |
| Elastase | | EC 3.4.21.14 | Novozyme | Bagsvaerd | Danemark |
| Trypsine | | EC 3.4.21 .36 | Novozyme | Bagsvaerd | Danemark |
| Alcalase | | EC 3.4.21.4 | Novozyme | Bagsvaerd | Danemark |
| Papaïne | Endopeptidases cystéine | EC 3.4.22.2 | Bio-Cat | Troy | Etats-Unis |
| Bromelaine (ananase) | | EC 3.4.22.32 | Bio-Cat | Troy | Etats-Unis |
| Prolyve NP | Endopeptidases aspartique | EC 3.4.23 | Lyven | Colombelles | France |
| Pepsine | | EC 3.4.23.1 | Sigma Aldrich | Saint-Quentin-Fallavier | France |
| Neutral protéase | Métallo-endopeptidase | EC 3.4.24.28 | Bio-Cat | Troy | Etats-Unis |
| Protex 50FP | Endopeptidase | EC 3.4.21 | Genencor International B.V. | Leiden | Pays-Bas |
| Pancrealyve | Exo & endo peptidase (cocktail protéases + amylases) | n.a.* | Lyven | Colombelles | France |
| Izyme BA | Protéase aspartique | EC 3.4.23 | Novozyme | Bagsvaerd | Danemark |
| Sumizyme | Cocktail enzymatique | n.a.* | Takabio - Shin Nihon | Aichi | Japon |
| Neutrase | Endoprotéase base de Zn de $\beta$ amylolique-faciens | EC 3.4.24 | Novozyme | Bagsvaerd | Danemark |
| Novozyme 37071 | Protéase | n.a.* | Novozyme | Bagsvaerd | Danemark |
| *n.a. : non applicable | | | | | |

Avantageusement, l'enzyme mise en œuvre lors de l'hydrolyse est une endopeptidases aspartique, telle que la Prolyve NP. Ce type d'enzyme permet d'obtenir de très bons résultats en terme de pureté de la chitine obtenue, en particulier lorsque ce type d'enzyme est appliqué à l'hydrolyse d'un gâteau de presse obtenu à partir de coléoptères et plus particulièrement de *T. molitor.*

L'enzyme ou le mélange d'enzymes est introduit(e) à une quantité allant de 0,2 à 10 % en poids de matière sèche estimé, préférentiellement de 0,4 à 8% en poids et plus préférentiellement de 0,5 à 2%. Par « poids de matière sèche estimé », on vise plus particulièrement le poids de matière sèche d'insectes ou de partie(s) d'insectes, tel qu'on peut l'estimer lors de l'entrée en étape d'hydrolyse enzymatique.

En termes d'activité enzymatique, la quantité d'enzyme ou de mélange d'enzymes introduite est équivalente à une activité comprise entre 2000 et 5000 SAPU (« *Spectrophotometric Acid Protease Unit* », décrit dans l'Exemple 5 ci-après), de préférence comprise entre 3000 et 4000 SAPU, pour 100 g en poids humide, avec une humidité de 30 à 70%, de substrat à transformer, c'est-à-dire de matière d'insectes ou de partie(s) d'insectes hydratée.

Avantageusement, l'étape d'hydrolyse enzymatique s'effectue en présence d'eau, telle que de l'eau douce. La quantité d'eau mise en œuvre lors de l'hydrolyse enzymatique est déterminée de la façon suivante : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1. On notera que ce ratio correspond également au ratio du poids d'eau sur le poids d'insecte, la masse volumique de l'eau étant de 1,0 g/mL dans les conditions normales de température et de pression.

Dans le procédé selon l'invention, l'hydrolyse enzymatique est précédée d'une étape de traitement des cuticules avec un agent oxydant.

De préférence, dans le procédé selon l'invention, l'agent oxydant mis en œuvre lors du traitement des cuticules est choisi parmi le groupe constitué par le peroxyde d'hydrogène, le permanganate de potassium, l'ozone et l'hypochlorite de sodium, plus préférentiellement encore, le peroxyde d'hydrogène.

Avantageusement, lorsque l'agent oxydant est le peroxyde d'hydrogène, la quantité introduite de cet agent pour le traitement des cuticules d'insectes est telle que la teneur en peroxyde d'hydrogène est comprise entre 1 et 33% en poids sur le poids total d'insectes, préférentiellement comprise entre 2 et 12% en poids sur le poids total d'insectes, préférentiellement de l'ordre de 6% en poids.

Préférentiellement, le traitement de cuticules d'insectes par l'agent oxydant s'effectue en présence d'eau, telle que de l'eau douce. Avantageusement, la quantité d'eau mise en œuvre lors du traitement des cuticules est déterminée de la façon suivante : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1.

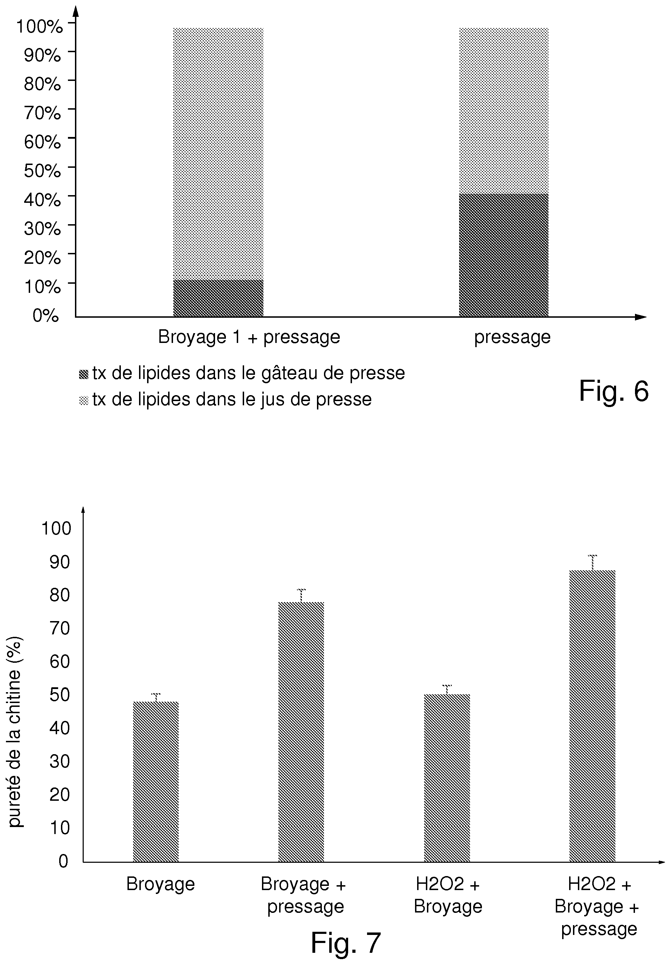

Le procédé selon l'invention permet l'obtention d'une chitine ayant un haut degré de pureté, tel qu'un degré de pureté (ou pureté gravimétrique) compris entre 55 et 95%, préférentiellement compris entre 60 et 95%, plus préférentiellement encore entre 70 et 90%, et encore plus préférentiellement entre 80 et 90% (voir l'Exemple 5 et la Figure 7).

De préférence, le procédé selon l'invention comporte une étape de broyage préalable à l'étape de pressage.

Cette étape de broyage a pour objectif de réduire les cuticules et/ou les insectes en particules afin de faciliter l'accès des enzymes au substrat lors de l'hydrolyse enzymatique. Cette étape permet également, lorsqu'elle est suivie d'une étape de pressage, de faciliter l'élimination du jus de presse et l'isolation de la matière solide.

Le broyage peut avantageusement être effectué avec un broyeur mixeur, tel qu'un broyeur mixeur à couteaux.

De préférence, à l'issue du broyage, la taille des particules d'insectes est inférieure à 1 cm (plus grande taille de particule observable à l'aide d'un microscope), de préférence inférieure à 0,5 cm, plus préférentiellement encore, une taille comprise entre 300 $\mu$m et 0,5 cm, plus préférentiellement entre 500 $\mu$m et 0,5 cm, et encore plus préférentiellement entre 500 $\mu$m et 1 mm.

Afin de faciliter le broyage, une quantité d'eau peut être ajoutée. Cette quantité d'eau est déterminée de la façon suivante : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1.

Avantageusement, dans le procédé selon l'invention, le traitement des cuticules avec un agent oxydant est effectué avant, concomitamment et/ou après l'étape de broyage et/ou de pressage.

Le procédé selon l'invention peut également comporter une étape d'abattage des insectes préalable à l'étape de pressage et/ou de broyage.

Cette étape d'abattage peut s'effectuer par des méthodes classiques d'élevage d'animaux à sang froid et/ou de petite taille (crustacés, poissons, escargots, etc.), tels que le froid (congélation), le chaud (ébouillantage), la privation d'oxygène, etc. Avantageusement, l'étape d'abattage des insectes est effectuée par ébouillantage. L'ébouillantage permet non seulement d'abattre les insectes, mais également d'abaisser la charge microbienne (réduction du risque d'altération et sanitaire) et d'inactiver les enzymes internes des insectes pouvant déclencher une autolyse, et ainsi un brunissement rapide de ceux-ci. Cet ébouillantage est réalisé de manière à provoquer la mort le plus rapidement possible, dans le respect du bien-être animal, et selon les préconisations scientifiques.

Alternativement, l'abattage peut être effectué par blanchiment. Le blanchiment présente les mêmes avantages cités ci-avant que l'ébouillantage.

Avantageusement, les insectes sont abattus, par exemple par ébouillantage ou blanchiment, puis broyés avant d'être pressés.

De préférence, l'étape d'ébouillantage est réalisée dans l'eau, telle que de l'eau douce, à une température de 95 à 105°C, préférentiellement de l'ordre de 100°C et pendant une durée de 2 à 20 min, préférentiellement, 5 à 15 min.

La quantité d'eau introduite à cette étape d'ébouillantage est déterminée de la façon suivante : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1.

Alternativement, l'étape de blanchiment est réalisée à la vapeur d'eau et/ou à l'eau à une température comprise entre 80 °C et 130 °C, de préférence entre 90°C et 120 °C.

Avantageusement, le traitement des cuticules avec un agent oxydant est effectué concomitamment et/ou après l'étape d'abattage et en particulier concomitamment et/ou après l'ébouillantage.

Plus particulièrement, le traitement des cuticules d'insectes avec l'agent oxydant peut être effectué pendant l'une ou plusieurs des étapes ci-après :

- concomitamment avec l'ébouillantage et/ou après l'étape d'ébouillantage, plus préférentiellement concomitamment avec l'ébouillantage, alternativement, concomitamment avec le blanchiment et/ou après l'étape de blanchiment, plus préférentiellement concomitamment avec le blanchiment. Plus particulièrement, lorsque le traitement des cuticules d'insectes est effectué pendant l'ébouillantage, l'agent oxydant peut être avantageusement ajouté à l'eau utilisée pour ébouillanter les insectes.
- avant, concomitamment et/ou après le broyage. Plus particulièrement, lorsque le traitement des cuticules d'insectes est effectué pendant le broyage, l'agent oxydant peut être avantageusement ajouté à l'eau utilisée pour le broyage.
- avant et/ou concomitamment avec le pressage.
- lors d'une étape spécifique de traitement des cuticules d'insectes.

Après l'hydrolyse enzymatique, le procédé selon l'invention peut comporter en outre une étape d'inactivation thermique visant à inactiver l'enzyme ou le mélange d'enzymes mis en œuvre lors de l'hydrolyse enzymatique.

A l'issue d'un procédé selon l'invention, la chitine peut être récupérée par pressage ou centrifugation du milieu de réaction de l'hydrolyse enzymatique. A ce stade, un co-produit d'intérêt de la chitine est également récupéré, à savoir un hydrolysat.

Par « hydrolysat», on désigne un produit qui comporte des protéines, des protéines hydrolysées, des peptides, des acides aminés et/ou d'autres composés dérivés d'une protéine, susceptibles d'être obtenus par hydrolyse enzymatique de protéines.

L' hydrolysat susceptible d'être obtenu comme co-produit de l'hydrolyse enzymatique par l'un quelconque des procédés selon l'invention présente au moins l'une quelconque des caractéristiques suivantes :

- Taux de cendres ≤3,5% en poids sur le poids total de matière sèche,
- Taux de cendres ≤2,5% lorsque l'hydrolysat est préparé à partir d'insectes végétariens,
- Taux de protéines hydrosolubles de taille >12400 g/mol, ≤20%, de préférence <18% en poids, sur le poids total de protéines hydrosolubles,
- Taux de protéines ≥70%, de préférence ≥74%,
- Taux de protéines ≥80% lorsque l'hydrolysat est préparé à partir d'insectes non volants,
- Taux de lipides ≤10% en poids sur le poids total de matière sèche,
- Taux de lipides ≤4,5% lorsque l'hydrolysat est préparé à partir d'insectes non volants,
- Digestibilité pepsique >93% par rapport au poids total de protéines,
- Taux d'abondance relative de au moins 5 quelconques acides aminés choisis parmi ASP, GLU, ALA, GLY, LEU, PRO, TYR, VAL, LYS ≥6%,
- Taux d'abondance relative de au moins 3 quelconques acides aminés choisis parmi ASP, GLU, ALA, LEU, PRO, TYR, VAL ≥8%.

Par « insecte végétarien », on vise un insecte qui n'a pas de protéines animales dans son régime alimentaire habituel. A titre d'exemple d'insecte végétarien, on peut citer les coléoptères, les lépidoptères ou les orthoptères.

Par « insecte volant », on vise un insecte qui est capable de voler à l'âge adulte, contrairement à un insecte dit « non volant ». A titre d'exemple d'insecte volant, on peut citer les lépidoptères ou les diptères. A titre d'exemple d'insecte non volant, on peut citer certains coléoptères ou les orthoptères.

Plus particulièrement, par « protéines hydrosolubles », on entend, parmi les protéines (ou protéines brutes), celles qui sont solubles dans une solution aqueuse dont le pH est compris entre 6 et 8, avantageusement entre 7,2 et 7,6.

De préférence, la solution aqueuse est une solution tampon dont le pH est compris entre 6 et 8, avantageusement entre 7,2 et 7,6.

Préférentiellement, la solution tampon est une solution phosphate NaCl, dont le pH est égale à 7,4 $\pm$ 0,2.

Plus particulièrement, la mesure de la taille des protéines hydrosolubles s'effectue selon le procédé ci-après : 100 mg d'échantillon sont placés dans 10 mL de tampon phosphate /NaCl (pH 7,4, 0,137 mM). L'échantillon a été agité durant une minute (vortex), centrifugé à 900 g durant 1 min et filtré ensuite sur une membrane de 0,45 $\mu$m. L'analyse a été réalisée sur un système de chromatographie par exclusion stérique, avec la colonne Nucleogel GFC-300, l'éluent utilisé est le tampon phosphate /NaCl (pH 7,4, 0,137 mM), le débit est de 1,0 mL/min, la détection est réalisée par un détecteur UV à 280 nm.

Avantageusement, l'hydrolysat comporte au moins 70%, de préférence au moins 74% de protéines, au maximum 3,5% de cendres et un taux de protéines hydrosolubles ayant une taille supérieure à 12400 g/mol, inférieur à 20%, préférentiellement inférieur à 18%.

De préférence, l'hydrolysat présente toutes les propriétés ci-avant

On notera en particulier que l'hydrolysat est caractérisé par sa teneur en glucosamine, et/ou l'un de ses dérivés (préférentiellement N-*acétyl*-glucosamine), plus particulièrement une teneur supérieure ou également à 0,01%, préférentiellement supérieure ou égale à 0,08% en poids sur le poids total de matière sèche de l'hydrolysat.

Toutes les unités et méthodes de mesure des caractéristiques indiquées ci-avant sont décrites dans les Exemples et, plus particulièrement, dans l'Exemple 6.

Cet hydrolysat peut avantageusement être complété avec des additifs pour équilibrer son profil nutritionnel afin d'être adapté à différents types d'animaux.

L'hydrolysat peut être concentré puis séché pour obtenir un hydrolysat séché. Alternativement, l'hydrolysat peut être sous forme liquide. Ces hydrolysats peuvent être utilisés comme un aliment ou un ingrédient alimentaire en particulier pour des animaux, ou, alternativement, ils peuvent être traités, par exemple, pour isoler des acides aminés.

Un mode de réalisation préférentiel d'un procédé selon l'invention est plus détaillé ci-après.

En particulier, ce mode de réalisation préférentiel décrit diverses étapes avantageuses pour un procédé selon l'invention, telles que des étapes de purification douce de la chitine : un second pressage, des lavages, filtration et séchage éventuels.

Enfin, la chitine étant généralement commercialisée sous forme de poudre, un second broyage peut également être réalisé. Celui-ci peut également être effectué afin de favoriser la réaction de désacétylation, qui permet de préparer du chitosan à partir de chitine. Les conditions de la réaction de désacétylation sont plus amplement décrites à l'étape 10 du mode de réalisation préférentiel détaillé ci-après.

La chitine susceptible d'être obtenue par le procédé selon l'invention présente au moins l'une quelconque des propriétés suivantes :

- Taux de cendres ≤3%, de préférence ≤2,5% en poids sur le poids total de matière sèche,
- Taux de cendres ≤1% lorsque la chitine est préparée à partir de *T. molitor,*
- Taux de lipides ≤6,5% en poids sur le poids total de matière sèche,
- Taux de lipides ≤2% lorsque la chitine est préparée à partir de *T. molitor,*
- Total des acides aminés ≤50%, de préférence ≤46%,
- Total des acides aminés ≤19% lorsque la chitine est préparée à partir d'insectes volants,
- Taux des acides aminés ≤23% lorsque la chitine est préparée à partir de *T. molitor* avec prolyve NP comme enzyme,
- Taux d'abondance relative de au moins 3 quelconque acides aminés, choisis parmi ALA, GLY, LEU, PRO, SER, TYR, VAL ≤10%,
- Taux d'abondance relative de LEU, PRO, VAL ≤10% lorsque la chitine est préparée à partir de *T. molitor,*
- Pureté colorimétrique ≥62%,
- Pureté par différence ≥40%, de préférence ≥46%,
- Pureté par différence ≥51% lorsque la chitine est préparée à partir de *T. molitor,*
- Pureté par différence ≥ 73% lorsque la chitine est préparée à partir d'insectes volants,
- Pureté par différence ≥73% lorsque la chitine est préparée à partir de *T. molitor* avec prolyve NP.

La chitine susceptible d'être obtenue par le procédé selon l'invention comporte avantageusement un taux d'acides aminés inférieur ou égal à 50%, préférentiellement inférieur ou égal à 46%, un taux de cendres inférieur ou égal à 3%, préférentiellement inférieur ou égal à 2,5%, et une pureté par différence supérieure ou égale à 40%, préférentiellement supérieure ou égale à 46%.

De préférence, la chitine présente toutes les propriétés ci-avant.

Toutes les unités et méthodes de mesure des caractéristiques indiquées ci-avant sont décrites dans les Exemples et, plus particulièrement, dans l'Exemple 6.

Un procédé particulièrement avantageux de production de chitine à partir d'insectes, comporte les étapes suivantes :

a) l'abattage des insectes,
b) le broyage des insectes,
c) le pressage des insectes,
d) l'hydrolyse enzymatique des cuticules d'insectes par une enzyme protéolytique,
e) la récupération de la chitine,

les cuticules d'insectes étant traitées avec un agent oxydant, avant l'étape d).

Les modes de réalisation préférés des diverses étapes a) à e) et du traitement des cuticules d'insectes avec un agent oxydant sont tels qu'indiqués ci-avant ou dans l'étape correspondante dans le mode de réalisation préférentiel ci-après.

Grâce aux conditions douces employées dans le procédé selon l'invention, la chitine susceptible d'être obtenue par ce procédé possède une structure proche de la chitine telle que présente à l'état naturel dans la cuticule de l'insecte tout en ayant un haut degré de pureté, tel qu'un degré de pureté compris entre 55 et 95%, préférentiellement compris entre 60 et 95%, plus préférentiellement encore entre 70 et 90%, et encore plus préférentiellement entre 80 et 90%.

Un procédé particulièrement avantageux de production de chitosan à partir d'insectes, comporte les étapes suivantes :

a) l'abattage des insectes,
b) le broyage des insectes,
c) le pressage des insectes,
d) l'hydrolyse enzymatique des cuticules d'insectes par une enzyme protéolytique,
e) la récupération de la chitine,
f) la désacétylation de la chitine récupérée,
g) la récupération du chitosan,

les cuticules d'insectes étant traitées avec un agent oxydant, avant l'étape d).

Les modes de réalisation préférés des diverses étapes a) à g) et du traitement des cuticules d'insectes avec un agent oxydant sont tels qu'indiqués ci-avant ou dans l'étape correspondante dans le mode de réalisation préférentiel ci-après.

La chitine et/ou le chitosan susceptible(s) d'être obtenu(s) par un procédé selon l'invention peu(ven)t avantageusement être utilisé(s) dans diverses applications :

- dans des compositions cosmétiques, pharmaceutiques, nutraceutiques ou diététiques,
- comme biomatériaux pour traiter des brûlures en tant que seconde peau, pour réaliser des pansements cornéens ou des fils chirurgicaux,
- comme agent filtrant, texturant, floculant et/ou adsorbant notamment pour la filtration et dépollution de l'eau.

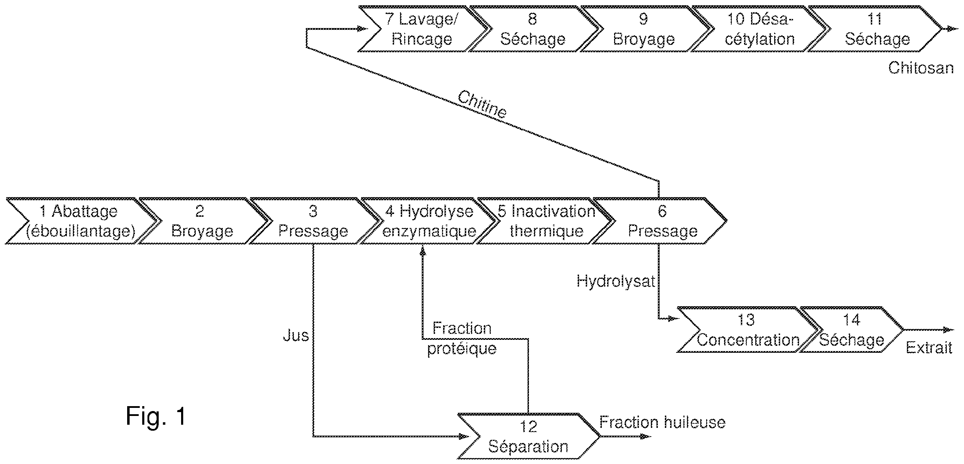

Selon un mode de réalisation préférentiel de l'invention, le procédé comporte les étapes suivantes, décrites schématiquement en Figure 1. On notera que certaines étapes sont indiquées comme facultatives dans ce mode de réalisation préférentiel.

### • Etape 1 : abattage des insectes

Cette étape 1 d'abattage permet d'abattre les insectes tout en abaissant la charge microbienne (réduction du risque d'altération et sanitaire) et en inactivant les enzymes internes des insectes pouvant déclencher une autolyse, et ainsi un brunissement rapide de ceux-ci.

L'abattage peut s'effectuer par ébouillantage.

Les insectes, de préférence des larves, sont ainsi ébouillantés à l'eau pendant 2 à 20 min, préférentiellement, 5 à 15 min. De préférence, l'eau est à température comprise entre 95 à 105 °C, préférentiellement 100 °C.

La quantité d'eau introduite à cette étape 1 d'ébouillantage est déterminée de la façon suivante : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1.

Alternativement, l'abattage peut s'effectuer par blanchiment. De préférence, les insectes sont blanchis à la vapeur (buses ou lit de vapeur) à une température comprise entre 80 et 130 °C, de préférence entre 90 et 120 °C, plus préférentiellement entre 95 et 105°C, encore plus préférentiellement 98°C ou bien à l'eau à une température comprise entre 95 et 105°C, préférentiellement 100 °C (par buses d'aspersion) ou en mode mixte (eau + vapeur) à une température comprise entre 80 et 130 °C, de préférence entre 90 et 120 °C, plus préférentiellement entre 95 et 105 °C. Le temps de séjour dans la chambre de blanchiment est compris entre 1 et 15 minutes, préférentiellement entre 3 et 7 min.

Lors de cette étape, il est également possible de traiter les cuticules d'insectes en utilisant une eau d'ébouillantage ou de blanchiment comportant l'agent oxydant selon les modalités indiquées dans l'étape intermédiaire ci-après.

**• Etape intermédiaire (facultative) : traitement des cuticules par l'agent oxydant**

Il est possible d'introduire dans le procédé une étape spécifique de traitement des cuticules par l'agent oxydant. Avantageusement, cette étape intermédiaire de traitement des cuticules s'effectue entre l'étape 1 d'abattage et l'étape 2 de broyage.

Cette étape intermédiaire s'effectue de préférence avec un agent oxydant choisi parmi le groupe constitué par le peroxyde d'hydrogène ($H_2O_2$), le permanganate de potassium ($KMnO_4$), l'ozone ($O_3$) et l'hypochlorite de sodium (NaClO), plus préférentiellement, le peroxyde d'hydrogène.

Selon un premier mode de réalisation, à l'issue de l'étape 1, lorsque celle-ci est faite par ébouillantage, on introduit directement dans la cuve d'ébouillantage l'agent oxydant, après éventuel refroidissement de l'eau d'ébouillantage à une température de l'ordre de 40 à 60°C, préférentiellement de l'ordre de 50°C.

Le peroxyde d'hydrogène tel que commercialisé est usuellement sous la forme d'une solution aqueuse, par exemple une solution à 30% en poids sur le poids total d'eau.

La quantité introduite de peroxyde d'hydrogène pour le traitement est telle que la teneur en peroxyde d'hydrogène est comprise entre 1 à 33% en poids sur le poids total d'insectes, préférentiellement, 2 à 12% en poids sur le poids total d'insectes, préférentiellement de l'ordre de 6% en poids.

Selon un second mode de réalisation, les insectes sont retirés de la cuve d'ébouillantage, tamisés et réintroduits dans une cuve.

Le peroxyde d'hydrogène est alors introduit dans la cuve sous la forme d'une solution aqueuse diluée, la teneur en peroxyde d'hydrogène étant alors comprise entre 1 et 33% en poids sur le poids d'eau, préférentiellement, 2 à 12% en poids sur le poids d'eau, préférentiellement de l'ordre de 6% en poids.

**• Etape 2 : broyage**

Les insectes sont retirés de la cuve d'ébouillantage ou de traitement ou de la chambre de blanchiment, ils sont ensuite tamisés, et placés dans un broyeur, tel qu'un broyeur mixeur à couteaux, permettant de réduire les insectes en particules.

Afin de faciliter le broyage, une quantité d'eau peut être ajoutée. Cette quantité d'eau est similaire à celle introduite lors de l'étape 1 d'ébouillantage : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1. Il est également possible de garder l'eau d'ébouillantage et/ou l'eau de l'étape intermédiaire pour effectuer cette étape. Cette eau est susceptible de contenir l'oxydant. Dans ce cas, le traitement des cuticules peut avoir lieu durant l'étape 1 d'ébouillantage et l'étape 2 de broyage ou pendant l'étape intermédiaire de traitement des cuticules et pendant l'étape de broyage.

De préférence, à l'issue du broyage, la taille des particules d'insectes est inférieure à 1 cm (plus grande taille de particule observable à l'aide d'un microscope), de préférence inférieure à 0,5 cm.

Préférentiellement, la taille des particules est comprise entre 300 $\mu$m et 0,5 cm, plus préférentiellement entre 500 $\mu$m et 0,5 cm, et encore plus préférentiellement entre 500 $\mu$m et 1 mm.

Il n'est pas nécessaire de réduire excessivement la taille des particules, par exemple à une taille inférieure à 250 $\mu$m.

Cette étape 2 de broyage favorise l'accès des enzymes à leur substrat.

Lors de cette étape, il est possible d'introduire dans le broyeur l'agent oxydant afin de traiter les cuticules selon les modalités indiquées dans l'étape intermédiaire ci-avant.

Lorsque le traitement des cuticules n'est pas effectué de manière concomitante avec le broyage, il est possible d'ajouter lors de cette étape, des additifs antioxydants communément employés pour la conservation et la stabilité du produit.

**• Etape 3 : pressage des cuticules d'insectes**

La pâte humide issue de l'étape 2 de broyage est ensuite placée dans une presse selon un mode opératoire qui permet de presser et séparer un jus comportant à la fois une fraction huileuse et une fraction protéique.

De préférence, l'étape de pressage permet d'obtenir un gâteau de presse comportant une teneur en huile inférieure ou égale à 20%, préférentiellement, inférieure ou égale à 15%, plus préférentiellement, inférieure ou égale à 12%, encore plus préférentiellement, inférieure ou égale à 10%.

De même, l'étape de pressage permet d'obtenir un gâteau de presse présentant une teneur en matière sèche comprise entre 30% et 60%, préférentiellement, comprise entre 40% et 55%, et plus préférentiellement comprise entre 45% et 50%.

Tout système de presse peut être utilisé pour réaliser l'étape de pressage, tel que par exemple, une presse mono-vis ou bi-vis (presse bi-vis de type Angel), un filtre-presse (filtre-presse de type Choquenet), une presse à plateaux, etc. Ces systèmes sont bien connus de l'homme du métier qui est a même de déterminer les conditions de pressage afin d'obtenir les teneurs en huile et/ou en eau mentionnées ci-avant.

Si la pâte humide issue de l'étape 2 de broyage a été obtenue avec une eau comportant l'oxydant, il peut être avantageux d'éliminer au moins une partie de cet oxydant avant l'étape 3 de pressage.

Cette étape 3 de pressage peut éventuellement être réalisée avant l'étape 2 de broyage à partir des insectes ébouillantés. Toutefois, il est avantageux d'effectuer l'étape 3 de pressage après l'étape 2 de broyage.

Cette étape 3 de pressage permet donc l'obtention d'un jus de presse et d'un gâteau de presse.

### • Etape 4 : hydrolyse enzymatique

La pâte humide issue de l'étape 2 de broyage ou le gâteau de presse issu de l'étape 3 de pressage est placé dans une cuve d'hydrolyse avec de l'eau.

Optionnellement, et comme cela sera décrit ci-après, la fraction protéique issue de l'étape 12 de séparation, peut être réintroduite dans cette étape 4 d'hydrolyse enzymatique, en la mélangeant au gâteau de presse.

De manière optionnelle et dans le cas où l'eau de l'ébouillantage ne contient pas d'oxydant, l'eau de l'ébouillantage peut être récupérée et réintroduite lors de l'étape d'hydrolyse. En effet, cette eau contient des fractions d'insectes solubilisées de par l'action de cet ébouillantage et l'utilisation de celle-ci lors de l'hydrolyse permet de réduire les pertes. Optionnellement, cette eau issue de l'ébouillantage peut être dégraissée, certaines cires pouvant s'être dissoutes dans l'eau.

La quantité d'eau introduite à cette étape 4 d'hydrolyse enzymatique est similaire à celle introduite lors de l'étape 1 d'ébouillantage : le ratio du volume d'eau en mL sur le poids en g d'insecte est de préférence compris entre 0,3 et 10, plus préférentiellement entre 0,5 et 5, encore plus préférentiellement entre 0,7 et 3, encore plus préférentiellement de l'ordre de 1.

L'hydrolyse enzymatique est réalisée avec une protéase, telle qu'une protéase commerciale pendant 4 à 8h, plus particulièrement pendant 4 à 5h, à un pH de 6 à 8, plus particulièrement de 6,5 à 7,5, à une température de 40 à 60 °C, plus particulièrement de 45 à 55 °C.

La quantité d'enzymes introduite lors de l'étape d'hydrolyse est inférieure à 10 % en poids sur le poids total de matière sèche estimé entrant en hydrolyse, préférentiellement inférieure à 6%, plus préférentiellement de l'ordre de 2 %. L'hydrolyse protéolytique engendre la production d'une phase soluble contenant les peptides, glucosamines et oligochitines et d'un résidu solide formé de chitine, principalement de copolymère chitine-polypeptides.

### • Etape 5 : inactivation thermique

Afin de stopper l'activité des enzymes de la réaction et de stabiliser la phase soluble de l'hydrolyse, on réalise une inactivation thermique en chauffant ce jus entre 80 et 105° pendant 10 à 25 min, préférentiellement, 15 à 20 minutes. Selon un mode opératoire, cette étape 5 d'inactivation thermique est réalisée selon les techniques habituelles de stérilisation de l'industrie agroalimentaire. Selon un autre mode opératoire, l'inactivation enzymatique est réalisée par chauffage sous rayonnement IR ou UV, ou micro-ondes.

### • Etape 6 : pressage

Le résidu solide, majoritairement composé de chitine, est récupéré puis pressé par un pressoir afin d'essorer au maximum ce résidu pour réinjecter dans la phase soluble ce pressât. Le résidu pressé ainsi formé est composé essentiellement de chitine, principalement sous la forme de copolymère chitine-polypeptides.

### • Etapes (facultatives) 7 et 8 : lavage et séchage

Le résidu solide est ensuite lavé, filtré, à nouveau lavé puis séché par les technologies classiques connues par l'homme de métier.

Avantageusement, le système de séchage est étudié pour protéger la structure du copolymère de chitine-polypeptides : l'hydrométrie, la ventilation et la composition de l'air sont contrôlés. Avantageusement, le séchage peut s'effectuer dans une étuve ventilée à une température de 60 à 80°C, préférentiellement de l'ordre de 70°C.

Optionnellement, ces étapes peuvent comporter une étape de délipidation terminale : le résidu solide est traité avec du HCl afin d'enlever les derniers résidus lipidiques, notamment les cires cuticulaires.

Les étapes 9 à 11 qui suivent, visent à transformer la chitine en chitosan et ne sont donc mises en œuvre que lorsque le produit souhaité est le chitosan.

### • Etape 9 (facultative) : broyage

Le résidu solide séché, comportant majoritairement de la chitine, est ensuite broyé, par exemple dans un broyeur à couteaux.

La production de chitosan à partir de chitine, par la réaction de désacétylation, dépend en grande partie de la taille des particules de chitine. Ainsi un broyage très fin du résidu solide séché avant désacétylation permet d'augmenter significativement les rendements et la vitesse de la réaction de désacétylation, comme cela est illustré dans le Tableau 1 ci-après :

**Tableau 1 :Efficacité de la désacétylation selon le broyage préalable de la chitine**

|  | Broyage 30 s | Broyage 45 s | Broyage 60 s | Broyage 120 s |
|---|---|---|---|---|
| 50% des particules | < 174 $\mu$m | < 117 $\mu$m | < 95 $\mu$m | < 67 $\mu$m |
| 90% des particules | < 310 $\mu$m | < 244 $\mu$m | < 157 $\mu$m | < 159 $\mu$m |
| DA* | 99% | 90% | 85% | 80% |
| *Mesure du Degré d'Acétylation DA | | | | |

Les conditions de la désacétylation effectuée dans l'essai rapporté dans le Tableau 1 sont les suivantes : 4h de réaction, 100°C, NaOH en solution aqueuse à 30% en volume, dans un ratio chitine estimée : solution de NaOH égal à 1:20.

Par conséquent, le résidu solide est préférentiellement broyé à une taille de particules inférieure à 200 $\mu$m, plus préférentiellement, inférieure à 160 $\mu$m.

### • Etape 10 : désacétylation

Le résidu solide, éventuellement broyé à l'étape 9, est ensuite placé dans un réacteur où est ajoutée une solution de soude concentrée pendant 4 à 24h, et préférentiellement 6 à 18h. L'hydroxyde de sodium en solution aqueuse à une teneur allant de 30% à 40% est ajouté selon un ratio poids en g de chitine broyée / volume en mL d'hydroxyde de sodium en solution aqueuse compris entre 1 :50 à 1 :10, de préférence de l'ordre de 1 :20. La cuve est ensuite chauffée, la température de désacétylation se situant entre 80 et 150 °C, de préférence entre 90 et 120 °C et plus préférentiellement à 100 °C.

On obtient ainsi du chitosan en poudre.

Le chitosan peut ensuite subir toute opération connue de l'homme du métier permettant de le fonctionnaliser, notamment par l'ajout de radicaux (carboxylation, hydroxylation...).

### • Etape 11 (facultative) : séchage

La poudre de chitosan est ensuite séchée entre 30 et 80°C, de préférence entre 50 et 70°C et de préférence à environ 60°C, afin d'obtenir une poudre ayant une teneur en matière sèche supérieure à 85%, plus particulièrement supérieure à 90%.

Les étapes qui suivent visent à récupérer une fraction huileuse et une fraction protéique à partir du jus obtenu à l'étape 3 de pressage et ne sont donc mises en œuvre que lorsque qu'une telle récupération est souhaitée.

### • Etape 12 : séparation

Le jus de presse subit une ou plusieurs étapes de séparation, afin de séparer la fraction huileuse (huiles des insectes) de la fraction protéique (protéines de l'hémolymphe des insectes). Ces étapes peuvent être réalisées par toute technologie de séparation des huiles bien connue de l'homme du métier, telle que la centrifugation, la décantation, la séparation par osmose inverse, l'ultrafiltration, le $CO_2$ supercritique, etc. ou une combinaison de plusieurs de ces technologies.

La séparation de la fraction huileuse peut être complexe, étant donné la présence d'huiles de compositions très différentes chez les insectes, les acides gras pouvant présenter de courtes chaines (C2-C5) ainsi que de très longues chaines (par exemple, pour les cires : > C25). La montée en température au-dessus du point de fusion de ces huiles (environ 38 °C) lors de la centrifugation permet de solubiliser cette crème et de faciliter la séparation de la fraction huileuse du reste du jus. Le centrifugat passe ensuite en décantation selon un mode opératoire (type décanteur ou tricanteur), afin de séparer au mieux les huiles et les protéines.

Ces étapes permettent ainsi l'obtention d'une fraction huileuse.

La fraction protéique, une fois séparée de la fraction huileuse, peut être mélangée au gâteau de presse issu de l'étape 3 de pressage juste avant l'étape 4 d'hydrolyse. En effet, souvent plus de 20% des protéines sont perdues dans le jus lors de l'étape 3 de pressage, d'où l'intérêt de récupérer cette fraction et de la soumettre à l'étape d'hydrolyse.

### • **Etape 13 (facultative) : concentration**

Selon un mode opératoire, la concentration est réalisée par évaporation sous vide de la partie aqueuse. Le concentrât présente un extrait sec supérieur à 10%, de préférence supérieur à 20%. Cette opération facilite le séchage et des additifs communément employés pour la conservation et la stabilité du produit peuvent être ajoutés à cette étape.

### • **Etape 14 (facultative) : séchage**

Le concentrât est enfin séché par des technologies connues de l'homme du métier, telles que par exemple, par pulvérisation / atomisation (« spray-drying »), qui permet d'obtenir un extrait, c'est-à-dire une poudre sèche de concentrât riche en peptides et glucosamines, les glucosamines étant en particulier issues de l'hydrolyse partielle de la chitine par $H_2O_2$ (essentiellement).

D'autres caractéristiques et avantages de l'invention apparaîtront dans les exemples qui suivent, donnés à titre illustratif, avec référence aux figures, qui représentent respectivement :

- La Figure 1 est un schéma d'un mode de réalisation préférentiel du procédé selon l'invention,
- La Figure 2 est un diagramme comparant le degré de pureté de la chitine obtenue par un procédé enzymatique avec et sans le peroxyde d'hydrogène,
- La Figure 3 est un diagramme illustrant l'influence du procédé d'extraction (enzymatique ou chimique) et du traitement avec un agent oxydant sur la chitine obtenue,
- La Figure 4 est un diagramme comparant le degré de pureté de la chitine obtenue par un procédé enzymatique comportant une ou plusieurs étapes préalables de broyage(s) et pressage,
- La Figure 5 est un diagramme comparant le taux de lipides mesuré dans différentes fractions du produit intermédiaire dont la chitine a été extraite,
- La Figure 6 illustre la répartition des lipides dans le jus et le gâteau de presse obtenus par un procédé enzymatique comportant des étapes préalables de broyage et de pressage ou une étape préalable de pressage,
- La Figure 7 est un diagramme comparant le degré de pureté de la chitine obtenue par un procédé enzymatique comportant une ou plusieurs étapes préalables de broyage(s), pressage et traitement avec un agent oxydant,
- La Figure 8 : Effet de la combinaison des étapes sur le taux de lipides de l'hydrolysat,
- La Figure 9 : Digestibilité de l'hydrolysat obtenu par le procédé,
- La Figure 10: TM + prolyve : abondance relative des acides aminés de l'hydrolysat,
- La Figure 11 : TM + sumizyme : abondance relative des acides aminés de l'hydrolysat,
- La Figure 12 : TM + novozyme : abondance relative des acides aminés de l'hydrolysat,
- La Figure 13 : TM + neutrase : abondance relative des acides aminés de l'hydrolysat,
- La Figure 14: HI + prolyve: abondance relative des acides aminés de l'hydrolysat,
- La Figure 15 : GM + prolyve : abondance relative des acides aminés de l'hydrolysat,
- La Figure 16 : AD + prolyve : abondance relative des acides aminés de l'hydrolysat,
- La Figure 17 : Effet de la combinaison de l'agent oxydant et de l'étape de pressage sur le taux de cendres dans la chitine,
- La Figure 18 : Synergie de la combinaison des étapes sur le taux de lipides dans la chitine,
- La Figure 19 : TM + prolyve : abondance relative des acides aminés de la chitine
- La Figure 20 : TM + sumizyme : abondance relative des acides aminés de la chitine,
- La Figure 21 : TM + novozyme : abondance relative des acides aminés de la chitine,
- La Figure 22 : TM + neutrase : abondance relative des acides aminés de la chitine
- La Figure 23 : HI + prolyve : abondance relative des acides aminés de la chitine,
- La Figure 24 : GM + prolyve : abondance relative des acides aminés de la chitine,
- La Figure 25 : AD + prolyve : abondance relative des acides aminés de la chitine,
- La Figure 26 : Pureté gravimétrique de la chitine - différentes enzymes,
- La Figure 27 : Pureté gravimétrique de la chitine - différents insectes,
- La Figure 28 : Pureté colorimétrique de la chitine obtenue à partir de *T. molitor* - différentes enzymes,

- La Figure 29 : Pureté colorimétrique de la chitine obtenue par le procédé - différents insectes,
- La Figure 30 : Pureté par différence de la chitine obtenue, et
- La Figure 31 : Degré de cristallinité des chitines obtenues.

Dans les exemples qui suivent, toute référence à un règlement ou à une directive concerne ledit règlement ou ladite directive tel qu'en vigueur à la date de dépôt de la présente demande.

**Exemple 1 : Exemple de procédé selon l'invention**

50 g de larves de *T. molitor* sont introduits dans un bêcher, placé dans un bain-marie à 100 °C et contenant 50 mL d'une solution de peroxyde d'hydrogène à 6% préalablement portée à ébullition. Après 5 minutes, le bêcher est retiré du bain-marie, les larves sont essorées, puis broyées avec un volume d'eau de 100 mL. Le liquide ainsi obtenu est passé dans une presse de type bi-vis, ce qui résulte en 17,89 $\pm$ 0,87 g de gâteau de presse avec un taux de matière sèche de 20,9 $\pm$ 1,3% et en 389,49 $\pm$ 6,53 g de jus de presse avec un taux de matière sèche de 4,04 $\pm$ 0,05%. 10 g (poids humide) de gâteau de presse ainsi obtenu sont transférés dans un Erlen Meyer contenant 50 mL d'eau et 0,1 g de protéase (Prolyve), l'ensemble est placé pendant 4 heures à 45 °C sous agitation magnétique (à un pH d'environ 6,5). L'Erlen Meyer est ensuite placé pendant 15 minutes dans un bain-marie à 90 °C afin de désactiver les enzymes, la solution est ensuite filtrée à 0,45-0,5 $\mu$m à chaud. La chitine ainsi obtenue est séchée pendant 24 heures à 70 °C. On obtient ainsi 0,64 $\pm$ 0,05 g de chitine par ce procédé.

**Exemple 2 : Influence de la présence du traitement des cuticules avec l'oxydant sur le degré de pureté de la chitine obtenue**

25 g de larves de *T. molitor* sont introduits dans un bêcher, placé dans un bain-marie à 100 °C et contenant 50 mL d'eau préalablement portée à ébullition. Après 5 minutes, le bêcher est retiré du bain-marie, les larves sont essorées, puis broyées avec un volume d'eau de 25 mL. Dans le cas de la réaction avec le peroxyde d'hydrogène, le liquide ainsi obtenu est placé pendant 1 heure en présence d'une solution de peroxyde d'hydrogène, puis transféré dans un Erlen Meyer de 250 mL contenant une solution de protéase (Sumizyme LP) à 4%, sinon, il est transféré directement dans l'Erlen Meyer contenant la solution de protéase. L'ensemble est placé pendant 4 heures à 45 °C sous agitation magnétique (pH approximativement à 6,5). L'Erlen Meyer est ensuite placé pendant 15 minutes dans un bain-marie à 90 °C afin de désactiver les enzymes, la solution est ensuite filtrée à 0,45-0,5 $\mu$m à chaud. La chitine ainsi obtenue est séchée pendant 24 heures à 70 °C.

Le résidu sec ainsi obtenu après utilisation du peroxyde d'hydrogène est de 6,3 $\pm$ 0,7 % par rapport à la matière sèche initiale, alors que le résidu sec résultant d'un procédé sans le peroxyde d'hydrogène est de 9,75 $\pm$ 0,9 % par rapport à la matière sèche initiale.

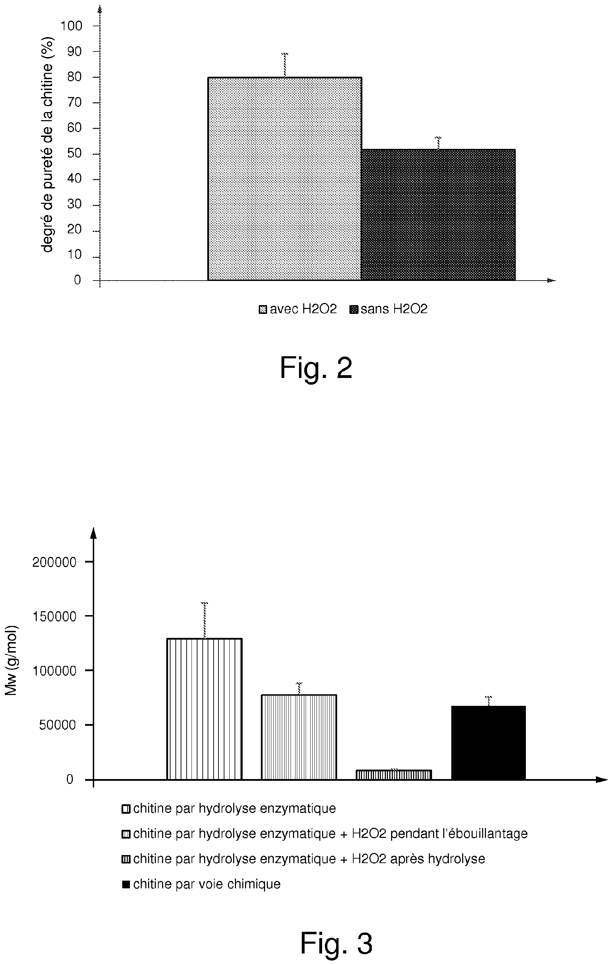

Le degré de pureté de la chitine est déterminé en comparaison de la masse en résidu sec obtenue par rapport à celle résultant d'une extraction chimique, 5 % de la matière sèche initiale. Il s'établit ainsi à 79,9 $\pm$ 9 % pour le produit obtenu après traitement avec le peroxyde et à 51,5 $\pm$ 4,9 % en l'absence de peroxyde (voir Figure 2).

**Exemple 3 : Influence de l'ordre de la séquence de réalisation du traitement des cuticules avec l'oxydant et de l'hydrolyse enzymatique**

Obtention de chitine par voie enzymatique (sans ajout d'oxydant)

50 g de larves de *T. molitor* sont introduits dans un bêcher, placé dans un bain-marie à 100 °C et contenant 50 mL d'eau préalablement portée à ébullition. Après 5 minutes, le bêcher est retiré du bain-marie, les larves sont essorées, puis broyées avec un volume d'eau de 100 mL. Le liquide ainsi obtenu est transféré dans un Erlen Meyer de 500 mL contenant 150 mL d'une solution de protéase (Prolyve) à 1%, l'ensemble est placé pendant 4 heures à 45 °C sous agitation magnétique (pH approximativement à 6,5). L'Erlen Meyer est ensuite placé pendant 15 minutes dans un bain-marie à 90 °C afin de désactiver les enzymes, la solution est ensuite filtrée à 0,45-0,5 $\mu$m à chaud. La chitine ainsi obtenue est séchée pendant 24 heures à 70 °C. On obtient ainsi 1,656 $\pm$ 0,021 g de chitine par ce procédé.

Obtention de chitine par voie enzymatique avec ajout de $H_2O_2$ pendant l'ébouillantage

50 g de larves de *T. molitor* sont introduits dans un bêcher, placé dans un bain-marie à 100 °C et contenant 50 mL d'une solution de $H_2O_2$ à 6% dans l'eau, préalablement portée à ébullition. Après 5 minutes, le bêcher est retiré du bain-marie, les larves sont essorées, puis mixées avec un volume d'eau de 100 mL. Le liquide ainsi obtenu est transféré dans un Erlen Meyer de 500 mL contenant 150 mL d'une solution de protéase (Prolyve) à 1%, l'ensemble est placé pendant 4 heures à 45 °C sous agitation magnétique. L'Erlen Meyer est ensuite placé pendant 15 minutes dans un bain-marie à 90 °C afin de désactiver les enzymes, la solution est ensuite filtrée à 0,45-0,5 μm à chaud. La chitine ainsi obtenue est séchée pendant 24 heures à 70 °C. On obtient ainsi 1,98 ± 0,22 g de chitine par ce procédé.

Obtention de chitine par voie enzymatique avec ajout de $H_2O_2$ après hydrolyse

50 g de larves de *T. molitor* sont introduits dans un bécher, placé dans un bain-marie à 100 °C et contenant 50 mL d'eau préalablement portée à ébullition. Après 5 minutes, le bécher est retiré du bain-marie, les larves sont essorées, puis broyées avec un volume d'eau de 100 mL. Le liquide ainsi obtenu est transféré dans un Erlen Meyer de 500 mL contenant 150 mL d'une solution de protéase (Prolyve) à 1%, l'ensemble est placé pendant 4 heures à 45 °C sous agitation magnétique (pH approximativement à 6,5). L'Erlen Meyer est ensuite placé pendant 15 minutes dans un bain-marie à 90 °C afin de désactiver les enzymes, la solution est ensuite filtrée à 0,45-0,5 μm à chaud. Le résidu est alors placé pendant 1 heure à 65 °C dans une solution de $H_2O_2$ à 6%. La chitine ainsi obtenue est filtrée (0,45-0,5 μm), puis séchée pendant 24 heures à 70 °C. On obtient ainsi 1,304 ± 0,091 g de chitine par ce procédé.

Obtention de chitine par voie chimique

50 g de larves de *T. molitor* sont introduits dans un bécher, placé dans un bain-marie à 100 °C et contenant 50 mL d'eau préalablement portée à ébullition. Après 5 minutes, le bécher est retiré du bain-marie, les larves sont essorées, puis broyées avec un volume d'eau de 60 mL. Le liquide ainsi obtenu est transféré avec 50 mL d'eau dans un flacon de 1 L. On y ajoute 500 mL de HCl 1M et on place l'ensemble sous agitation pendant 1 heure à 90 °C. Le milieu réactionnel est ensuite filtré et lavé avec de l'eau jusqu'à l'obtention d'un résidu clair. Ce résidu est ensuite transféré dans un flacon de 1 L auquel on ajoute 500 mL de NaOH 1M et on maintient le tout sous agitation à 90 °C pendant 24 heures. Le milieu réactionnel est alors filtré et lavé jusqu'à l'obtention d'un filtrat clair, le résidu est finalement séché 24 heures à 70 °C. On obtient ainsi 0,944 ± 0,005 g de chitine par ce procédé.

Détermination (par viscosimétrie) de la masse moléculaire de la chitine obtenue

Un flacon contenant 1 g de chitine et 10 mL de NaOH 1M est placé pendant 4 heures à 90 °C. Le mélange est ensuite filtré (0,45-0,5 μm) et le résidu ainsi lavé est placé 24 heures à 70 °C.

La préparation du solvant: 5 g de LiCl sont placés dans 100 mL de N,N-diméthylacétamide, sous agitation et pendant 4-5 heures (jusqu'à complète dissolution).

La solution-mère est obtenue en dissolvant 0,2 mg de chitine dans 1 mL de solvant. A partir de cette solution-mère, des solutions-filles, avec des concentrations de 0,1 mg/mL, 0,08 mg/mL et 0,04 mg/mL, sont préparées. La viscosité de ces différentes solutions est ensuite mesurée en triplicata avec un viscosimètre de type Ostwald et la masse moléculaire est calculée suivant la formule :

$$[\eta]= KM_w^{\alpha} \quad (1)$$

avec

[η] : viscosimétrie intrinsèque en cm³/g,
$M_w$ : masse molaire de la chitine en g/mol (ou Da), et
les coefficients de Mark-Houvink $\alpha$ = 0,71 et K = 0,000893, la viscosité intrinsèque étant obtenue selon :

$$[\eta]=\eta_r/C \quad (2)$$

avec

$\eta_r$ : viscosité réduite (sans unités),
C : concentration en mg/mL,

la viscosité réduite étant obtenue selon :

$$\eta_r = t/t_0 \quad (3)$$

avec

t : le temps de chute mesuré pour la solution en s,

$t_0$ : le temps de chute mesuré pour le solvant en s.

Il peut être observé sur la Figure 3 que la taille de la molécule de la chitine obtenue est fonction de la méthode d'extraction utilisée. Ainsi, la méthode chimique endommage l'intégrité de la molécule ($M_w$ obtenue est inférieure à 70000 g/mol), mais le traitement le plus drastique est celui qui consiste à blanchir la chitine par le peroxyde d'hydrogène après hydrolyse, même enzymatique ($M_w$ inférieure à 9000 g/mol).

Le procédé selon l'invention (hydrolyse enzymatique avec l'ajout du peroxyde d'hydrogène pendant ou juste après l'ébouillantage, c'est-à-dire en début du procédé), certes, diminue la taille de la molécule par rapport à celle que l'on peut trouver initialement dans l'insecte ($M_w$ de la chitine par hydrolyse enzymatique simple est de 130000 g/mol), mais dans une bien moindre mesure ($M_w$ de près de 80000 g/mol) et le résultat obtenu est supérieur à celui lié à l'extraction chimique traditionnelle.

## Exemple 4 : Influence de l'étape de pressage préalable à l'hydrolyse enzymatique sur le degré de pureté de la chitine obtenue

Différents types de prétraitement mécanique ont été testés, le broyage (« broyage 1 ») seul, le broyage suivi du pressage, le broyage suivi du pressage et d'un second broyage (« broyage 2 »), ainsi que le pressage seul.

Pour le pressage, une presse de type Angel a été utilisée, dans les conditions suivantes :

- Vitesse = 82 tr/min;
- W (énergie) = 3 HP (chevaux vapeur) soit 2,68 x $10^6$ J;
- Porosité (approximative) = 0,5 mm dans la première partie et 0,2 mm dans la dernière partie.

### 1. Matériel et méthodes

#### Production de chitine avec un broyage

200 g de larves de *T. molitor* sont introduits dans un bécher, placé dans un bain-marie à 100 °C et contenant 200 mL d'eau préalablement portée à ébullition. Après 5 minutes, le bécher est retiré du bain-marie, les larves sont essorées, puis mixées avec un volume d'eau de 200 mL. Le liquide ainsi obtenu est transféré dans un Erlen Meyer contenant 2 g de protéase (Prolyve), l'ensemble est placé pendant 4 heures à 45 °C sous agitation magnétique (à un pH d'environ 6,5). L'Erlen Meyer est ensuite placé pendant 15 minutes dans un bain-marie à 90 °C afin de désactiver les enzymes, la solution est ensuite filtrée à 0,45-0,5 µm à chaud. La chitine ainsi obtenue est séchée pendant 24 heures à 70 °C. On obtient ainsi 8,13 ± 0,27 g de chitine par ce procédé.

#### Production de chitine avec un broyage suivi d'un pressage

200 g de larves de *T. molitor* sont introduits dans un bécher, placé dans un bain-marie à 100 °C et contenant 200 mL d'eau préalablement portée à ébullition. Après 5 minutes, le bécher est retiré du bain-marie, les larves sont essorées, puis mixées avec un volume d'eau de 200 mL. Le liquide ainsi obtenu est passé dans une presse de type bi-vis. 30 g du gâteau de presse ainsi obtenu sont transférés dans un Erlen Meyer contenant 150 mL d'eau et 0,3 g de protéase (Prolyve), l'ensemble est placé pendant 4 heures à 45 °C sous agitation magnétique (à un pH d'environ 6,5). L'Erlen Meyer est ensuite placé pendant 15 minutes dans un bain-marie à 90 °C afin de désactiver les enzymes, la solution est ensuite filtrée à 0,45-0,5 µm à chaud. La chitine ainsi obtenue est séchée pendant 24 heures à 70 °C. On obtient ainsi 4,71 ± 0,11 g de chitine par ce procédé.

#### Production de chitine avec un premier broyage (« broyage 1 ») suivi d'un pressage et d'un second broyage (« broyage 2 »)

200 g de larves de *T. molitor* sont introduits dans un bécher, placé dans un bain-marie à 100 °C et contenant 200 mL d'eau préalablement portée à ébullition. Après 5 minutes, le bécher est retiré du bain-marie, les larves sont essorées, puis mixées avec un volume d'eau de 200 mL. Le liquide ainsi obtenu est passé dans une presse de type bi-vis. Le gâteau de presse ainsi obtenu est séché 24 heures dans une étuve à 70 °C, puis broyé à 250 µm. 10 g de la poudre ainsi obtenue sont transférés dans un Erlen Meyer contenant 50 mL d'eau et 0,1 g de protéase (Prolyve), l'ensemble est placé pendant 4 heures à 45 °C sous agitation magnétique (à un pH d'environ 6,5). L'Erlen Meyer est ensuite placé pendant 15 minutes dans un bain-marie à 90 °C afin de désactiver les enzymes, la solution est ensuite filtrée à 0,45-0,5 $\mu$m à chaud. La chitine ainsi obtenue est séchée pendant 24 heures à 70 °C. On obtient ainsi 4,93 ± 0,12 g de chitine par ce procédé.

Production de chitine avec pressage uniquement

200 g de larves de *T. molitor* sont introduits dans un bécher, placé dans un bain-marie à 100 °C et contenant 200 mL d'eau préalablement portée à ébullition. Après 5 minutes, le bécher est retiré du bain-marie, les larves sont essorées, puis passées dans une presse de type bi-vis. 90 g de gâteau de presse ainsi obtenu sont transférés dans un Erlen Meyer contenant 450 mL d'eau et 0,9 g de protéase (Prolyve), l'ensemble est placé pendant 4 heures à 45 °C sous agitation magnétique (à un pH d'environ 6,5). L'Erlen Meyer est ensuite placé pendant 15 minutes dans un bain-marie à 90 °C afin de désactiver les enzymes, la solution est ensuite filtrée à 0,45-0,5 $\mu$m à chaud. La chitine ainsi obtenue est séchée pendant 24 heures à 70 °C. On obtient ainsi 6,48 ± 0,28 g de chitine par ce procédé.

Production de chitine par voie chimique

50 g de larves de *T. molitor* sont introduits dans un bécher, placé dans un bain-marie à 100 °C et contenant 50 mL d'eau, préalablement portée à ébullition. Après 5 minutes, le bécher est retiré du bain-marie, les larves sont essorées, puis mixées avec un volume d'eau de 60 mL. Le liquide ainsi obtenu est transféré dans un récipient de 1 L et 500 mL d'une solution d'HCl 1M sont ajoutés. L'ensemble est placé à 90 °C sous agitation pendant 1 heure. Le contenu est ensuite filtré et le résidu solide est transféré dans un flacon de 1 L contenant 500 mL d'une solution de NaOH 1M, l'ensemble est placé à 90 °C sous agitation pendant 24 heures. Le résidu est ensuite filtré et placé en étuve ventilée à 70 °C pendant 24 heures. On obtient ainsi 0,944 ± 0,005 g de chitine purifiée par voie chimique.

Calcul du degré de pureté

Le degré de pureté de la chitine est déterminé en comparaison de la masse en résidu sec obtenu par rapport à celle résultant d'une extraction chimique, 5 % environ de la matière sèche initiale.

Mesure du taux de lipides

On place 2 g d'échantillon dans un bécher, on y ajoute 0,2 g de $Na_2SO_4$ et 15 mL de $CHCl_3$/MeOH (2/1 v/v). L'ensemble est placé sous agitation magnétique pendant 20 minutes, ensuite la solution est filtrée, le résidu est placé de nouveau dans le bécher avec 10 mL de $CHCl_3$/MeOH (2/1 v/v). L'ensemble est placé sous agitation magnétique pendant 15 minutes, ensuite la solution est filtrée, les phases solvants sont réunies et évaporées à poids constant. La teneur en lipides est déterminée comme pourcentage de masse après extraction-évaporation par rapport à la masse initiale de l'échantillon (2 g).

2. Résultats

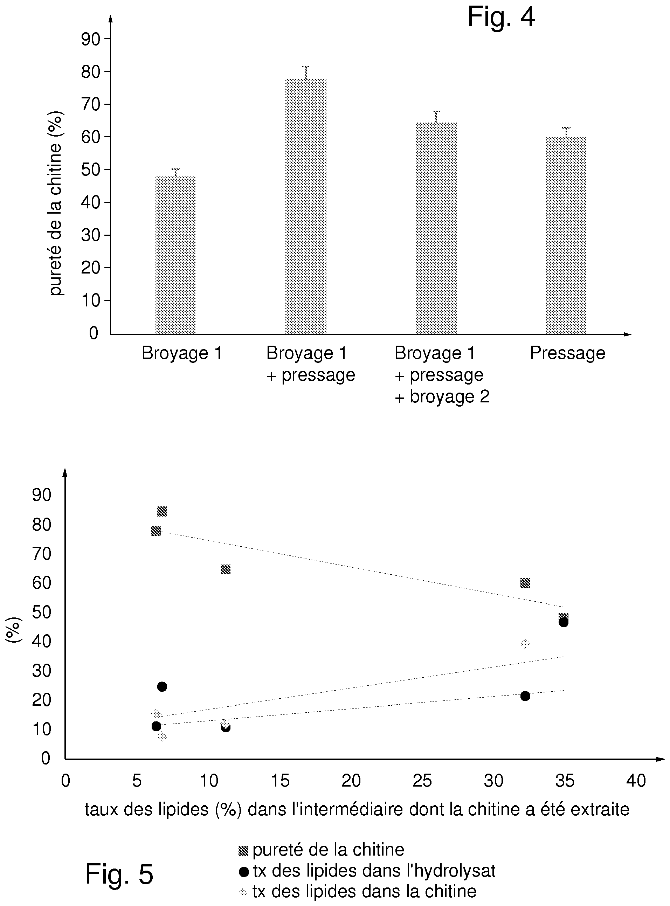

Comme cela peut être constaté sur la Figure 4, le procédé a une influence sur la pureté de la chitine obtenue, les meilleurs résultats étant obtenus avec un pressage a minima. Le meilleur résultat est obtenu avec le broyage suivi du pressage, à savoir une chitine présentant un degré de pureté de 78% et le moins bon avec le broyage seul, à savoir une chitine présentant un degré de pureté de 48%.

Une analyse plus fine du produit intermédiaire dont la chitine a été extraite, permet de constater qu'un bas taux de lipides est favorable à une plus grande pureté de la chitine obtenue (Figure 5). Par « produit intermédiaire », on vise le produit entrant en hydrolyse enzymatique, c'est-à-dire issu de la dernière étape avant hydrolyse, à savoir, selon les procédés de production ci-avant, l'étape de broyage 1 ou 2 ou l'étape de pressage.

L'analyse du taux des lipides dans la chitine et le jus d'hydrolyse permet en effet de relever qu'en fonction du taux de lipides initiaux présents dans le produit intermédiaire, le taux des lipides dans la chitine est relativement stable, de 7 à 15%, tandis que le taux des lipides dans l'hydrolysat varie, quant à lui, de 11 à 47% (Figure 5).

Plus particulièrement, si le produit intermédiaire a une teneur en lipides de 35%, alors :

- la chitine qui résultera de l'hydrolyse ne sera pure qu'à 50% et comportera 10% de lipides, et
- la teneur en lipides de l'hydrolysat avoisinera quant à elle les 40%.

En revanche, si la teneur en lipides du produit intermédiaire est de 7%, alors :

- la chitine obtenue après hydrolyse aura une pureté de 80% et aura une teneur en lipides de 8% et l'hydrolysat présentera également une faible teneur en lipides, de l'ordre de 10%.

Cela indique que lorsque le taux des lipides initiaux est élevé, supérieur à 12%, l'enzyme responsable d'hydrolyse est amenée à hydrolyser non seulement les protéines, mais également les lipides par promiscuité catalytique. C'est ainsi qu'un taux de lipides dans la chitine similaire est obtenu à savoir 8,6 et 7,9% dans les cas où les lipides initiaux étaient de 35 et 7% respectivement. En revanche, la pureté de la chitine passe dans ce cas de 48 à 84% respectivement. Ainsi, sur les 52% d'impuretés d'une part et 16% de l'autre, 8% en moyenne sont dus aux lipides, la quantité de protéines restées accrochées à la chitine est donc de 38 points supérieure dans le cas où davantage de lipides étaient présents dans le produit intermédiaire soumis à l'hydrolyse.

Finalement, l'importance du broyage en amont du pressage peut également être étudiée (Figure 6). Il apparait ainsi clairement que la répartition des lipides entre le gâteau et le jus de presse est bien plus efficace, 12,9 *versus* 87,1 contre 42,7 *versus* 57,3, lorsqu' un broyage préalable a été réalisé.

**Exemple 5 : Influence de la combinaison d'étapes préalables à l'hydrolyse enzymatique sur le degré de pureté de la chitine obtenue**

Différents types de prétraitement ont été testés, le broyage seul, le broyage suivi du pressage, le traitement des cuticules avec un agent oxydant ($H_2O_2$) suivi d'un broyage et le traitement des cuticules avec un agent oxydant ($H_2O_2$) suivi d'un broyage et d'un pressage.

Pour le pressage, la presse de type Angel décrite à l'Exemple 4 a été utilisée dans les conditions suivantes :

- Vitesse = 82 tr/min;
- W (énergie) = 3 HP (chevaux vapeur) soit 2,68 x $10^6$ J;
- Porosité (approximative) = 0,5 mm dans la première partie et 0,2 mm dans la dernière partie.

1. Matériel et méthodes

Production de chitine avec un broyage

200 g de larves de *T. molitor* sont introduits dans un bécher, placé dans un bain-marie à 100 °C et contenant 200 mL d'eau préalablement portée à ébullition. Après 5 minutes, le bécher est retiré du bain-marie, les larves sont essorées, puis mixées avec un volume d'eau de 200 mL. Le liquide ainsi obtenu est transféré dans un Erlen Meyer contenant 2 g de protéase (Prolyve), l'ensemble est placé pendant 4 heures à 45 °C sous agitation magnétique (à un pH d'environ 6,5). L'Erlen Meyer est ensuite placé pendant 15 minutes dans un bain-marie à 90 °C afin de désactiver les enzymes, la solution est ensuite filtrée à 0,45-0,5 μm à chaud. La chitine ainsi obtenue est séchée pendant 24 heures à 70 °C. On obtient ainsi 8,13 ± 0,27 g de chitine par ce procédé.

Production de chitine avec un broyage suivi d'un pressage

200 g de larves de *T. molitor* sont introduits dans un bécher, placé dans un bain-marie à 100 °C et contenant 200 mL d'eau préalablement portée à ébullition. Après 5 minutes, le bécher est retiré du bain-marie, les larves sont essorées, puis broyées avec un volume d'eau de 200 mL. Le liquide ainsi obtenu est passé dans une presse de type bi-vis. 30 g du gâteau de presse ainsi obtenu sont transférés dans un Erlen Meyer contenant 150 mL d'eau et 0,3 g de protéase (Prolyve), l'ensemble est placé pendant 4 heures à 45 °C sous agitation magnétique (à un pH d'environ 6,5). L'Erlen Meyer est ensuite placé pendant 15 minutes dans un bain-marie à 90 °C afin de désactiver les enzymes, la solution est ensuite filtrée à 0,45-0,5 μm à chaud. La chitine ainsi obtenue est séchée pendant 24 heures à 70 °C. On obtient ainsi 4,71 ± 0,11 g de chitine par ce procédé.

Production de chitine avec un traitement des cuticules avec un agent oxydant ($H_2Q_2$) suivi d'un broyage

50 g de larves de *T. molitor* sont introduits dans un bécher, placé dans un bain-marie à 100 °C et contenant 50 mL d'une solution de peroxyde d'hydrogène à 6% préalablement portée à ébullition. Après 5 minutes, le bécher est retiré du bain-marie, les larves sont essorées, puis broyées avec un volume d'eau de 100 mL. Le liquide ainsi obtenu est transféré dans un Erlen Meyer contenant 150 mL d'eau et 0,5 g de protéase (Prolyve), l'ensemble est placé pendant 4 heures à 45 °C sous agitation magnétique (à un pH d'environ 6,5). L'Erlen Meyer est ensuite placé pendant 15 minutes dans un bain-marie à 90 °C afin de désactiver les enzymes, la solution est ensuite filtrée à 0,45-0,5 μm à chaud. La chitine ainsi obtenue est séchée pendant 24 heures à 70 °C. On obtient ainsi 1,98 ± 0,22 g de chitine par ce procédé.

Production de chitine avec un traitement des cuticules avec un agent oxydant ($H_2HO_2$) suivi d'un broyage et d'un pressage

Cet essai est réalisé selon l'Exemple 1.

Calcul du degré de pureté

La pureté de chitine est calculée par gravimétrie en fonction du taux de chitine, initialement contenu dans les larves, *i.e.* 5% environ du poids sec des larves.

2. Résultats

Les résultats sont présentés en Figure 7. La pureté de la chitine obtenue par hydrolyse enzymatique dépend fortement du procédé de prétraitement utilisé. Ainsi, lorsque seul un broyage est réalisé avant l'hydrolyse enzymatique, ce procédé ne donne lieu qu'à une pureté de 48%, alors que l'adjonction d'une étape de pressage ou d'un traitement des cuticules avec un agent oxydant permet d'obtenir une pureté de 50-78%. Enfin, la combinaison d'une étape de pressage avec le traitement cuticules avec un agent oxydant permet d'atteindre une chitine présentant un degré de pureté de 88%.

Effet de synergie résultant de la combinaison d'une étape de pressage avec une étape de traitement des cuticules avec un agent oxydant

Le Tableau 2 ci-dessous reprend les résultats présentés en Figure 7.

**Tableau 2 : Amélioration de la pureté de la chitine selon le procédé de prétraitement utilisé**

|  | Broyage seul | Broyage + pressage | H2O2 + Broyage | H2O2 + broyage + pressage |
|---|---|---|---|---|
| Pureté de la chitine obtenue | 48% | 78% | 50% | 88% |
| Amélioration de la pureté de la chitine par rapport au broyage seul | - | + 63% | + 4% | + 83% |

Le Tableau 2 met en évidence l'effet synergique de la combinaison d'une étape de pressage et d'une étape de traitement avec un agent oxydant.

**Exemple 6** : **Caractérisation de l'hydrolysat et de la chitine selon le procédé de production mis en œuvre**

**I. Matériel et méthodes**

a) Matériel

Insectes

Différents insectes ont été étudiés :

- un coléoptère : *Tenebrio molitor (T. molitor* ou TM),
- un lépidoptère : *Galleria mellonella (G. melonella* ou GM),
- un diptère : *Hermetia illucens (H. illucens* ou HI), et
- un orthoptère : *Acheta domesticus (A. domesticus* ou AD).

Enzymes

Différentes enzymes ont été mises en œuvre lors de l'étape d'hydrolyse.
Cette mesure de l'activité enzymatique est basée sur le principe de la mesure de libération de la tyrosine à 275 nm lors de l'hydrolyse de la caséine par une enzyme protéolytique (Valley research SAPU Assay method, par Karen PRATT).

$$\frac{SAPU}{g} = \frac{(\Delta A - i) \times 11}{m \times 30 \times C \times 1}$$

SAPU/g = une unité spectrophotométrique de protéase

$\Delta A$ = absorbance corrélée

i = ordonnée à l'origine

11 = volume final de réaction

M = coefficient directeur de la courbe de calibration

30 = temps de réaction (en minutes)

C = concentration de l'enzyme (g/mL) dans la solution enzymatique ajoutée

1 = 1 mL volume de la solution d'enzyme ajoutée

La courbe de calibration est obtenue par mesure de l'absorbance des solutions de tyrosine de différentes concentrations dans un tampon phosphate (0,2 M, pH 7).

5 mL d'une solution de caséine (0,7% m/v, tampon phosphate 0,2 M, pH 7, chauffée pendant 30 minutes à 90 °C et additionnée de 3,75 g/L$_{solution}$) sont incubés avec 1 mL de la solution enzymatique (0,15 g dans 100 mL du tampon glycine, 0,05M) à tester à 37 °C pendant 30 minutes. On y ajoute ensuite 5 mL d'une solution de TCA (18 g TCA, 11,35 g d'acétate de sodium anhydre, 21 mL d'acide acétique glacial, complétée avec l'eau déminéralisée à 1 litre de solution), mélange sur un vortex, filtre et mesure l'absorbance à 275 nm.

Le blanc est préparé de façon identique mais sans ajout d'enzyme, 1 mL d'eau déminéralisée est ajouté à la place afin de retrouver le même volume réactionnel.

Les activités ainsi mesurées pour les différentes enzymes utilisées (Prolyve NP, Novozyme 37071, Neutrase et Sumizyne) sont répertoriées dans le Tableau 3.

| enzyme | Prolyve | Novozyme | Neutrase | Sumizyme |
|---|---|---|---|---|
| Activité enzymatique voulue | 3789,52 | 3789,52 | 3789,52 | 3789,52 |
| Activité enzymatique / g | 3789,52 | 1662,35 | 2213,24 | 3237,19 |
| m (g) | 1,00 | 2,28 | 1,71 | 1,17 |

**Tableau 3.** Correspondance des activités et masses enzymatiques des enzymes utilisées

b) Procédés de production

Procédé de production avec broyage seul (désigné « broyage » dans les figures)

600 g d'insectes (qu'il s'agisse de larves dans le cas de *T. molitor, G. melonella* ou *H. illucens,* ou de criquets dans le cas de *A. domesticus*) frais sont introduits dans une enceinte où ils sont abattus à la vapeur d'eau (115 °C, 5 minutes). Les insectes sont ensuite introduits dans un appareil de mixage et 75 mL d'eau pour 100 g d'insectes sont ajoutés, l'ensemble est ensuite mixé. 100 g (poids humide) de produit ainsi obtenu sont ensuite introduits dans un ballon tricol muni d'un réfrigérant et d'un agitateur mécanique, une enzyme protéolytique d'une activité équivalente à 3789 SAPU est alors ajouté. La réaction est ensuite portée à 45 °C pendant 4 heures. La température est ensuite portée à 90 °C pendant 15 minutes, le mélange réactionnel est enfin filtré (0,40 - 0,45 μm). Le résidu est séché pendant 24 heures à 70 °C : il s'agit donc de la chitine obtenue par voie enzymatique de purification ; le filtrat est congelé et est lyophilisé : il s'agit donc de l'hydrolysat.

Le procédé est identique quel que soit l'insecte ou l'enzyme étudié.

Procédé de production avec broyage suivi d'un pressage (désigné « broyage +pressage » dans les figures)

600 g d'insectes (qu'il s'agisse de larves dans le cas de *T. molitor, G. melonella* ou *H. illucens,* ou de criquets dans le cas de *A. domesticus*) frais sont introduits dans une enceinte où ils sont abattus à la vapeur d'eau (115 °C, 5 minutes). Les insectes sont ensuite introduits dans un appareil de mixage et 75 mL d'eau pour 100 g d'insectes sont ajoutés, l'ensemble est ensuite mixé et pressé (presse bi-vis, ou filtre presse, ou autre système de pressage). 100 g (poids humide) de produit ainsi obtenus sont ensuite introduits dans un ballon tricol muni d'un réfrigérant et d'un agitateur magnétique, 500 mL d'eau sont ajoutés ainsi qu'une enzyme protéolytique d'une activité équivalente à 3789 SAPU. La réaction est ensuite portée à 45 °C pendant 4 heures. La température est ensuite portée à 90 °C pendant 15 minutes, le mélange réactionnel est enfin filtré (0,40 - 0,45 $\mu$m). Le résidu est séché pendant 24 heures à 70 °C : il s'agit donc de la chitine obtenue par voie enzymatique de purification ; le filtrat est congelé et est lyophilisé : il s'agit donc de l'hydrolysat.

Le procédé est identique quel que soit l'insecte ou l'enzyme étudiés.

Procédé de production avec broyage et traitement oxydant des cuticules d'insectes (désigné « broyage + H2O2 » dans les figures)

600 g d'insectes (qu'il s'agisse de larves dans le cas de *T. molitor, G. melonella* ou *H. illucens,* ou de criquets dans le cas de *A. domesticus*) frais sont introduits dans une enceinte où ils sont abattus à la vapeur d'un mélange eau/H$_2$O$_2$ (6%) (115 °C, 5 minutes). Les insectes sont ensuite introduits dans un appareil de mixage et 75 mL d'eau par 100 g d'insectes sont ajoutés, l'ensemble est ensuite mixé. 100 g (poids humide) de produit ainsi obtenu sont ensuite introduits dans un ballon tricol muni d'un réfrigérant et d'un agitateur mécanique, une enzyme protéolytique d'une activité équivalente à 3789 SAPU est alors ajoutée. La réaction est ensuite portée à 45 °C pendant 4 heures. La température est ensuite portée à 90 °C pendant 15 minutes, le mélange réactionnel est enfin filtré (0,40 - 0,45 $\mu$m). Le résidu est séché pendant 24 heures à 70 °C : il s'agit donc de la chitine obtenue par voie enzymatique de purification ; le filtrat est congelé et est lyophilisé : il s'agit donc de l'hydrolysat.

Le procédé est identique quel que soit l'insecte ou l'enzyme étudiés.

Procédé de production avec broyage, traitement oxydant des cuticules d'insectes et pressage (désigné « broyage + H2O2 + pressage » dans les figures)

600 g d'insectes (qu'il s'agisse de larves dans le cas de *T. molitor, G. melonella* ou *H. illucens,* ou de criquets dans le cas de *A. domesticus*) frais sont introduite dans une enceinte où ils sont abattus à la vapeur d'un mélange eau/H$_2$O$_2$ (115 °C, 5 minutes). Les insectes sont ensuite introduits dans un appareil de mixage et 75 mL d'eau par 100 g d'insectes sont ajoutés, l'ensemble est ensuite mixé et pressé (presse bi-vis, ou filtre presse, ou autre système de pressage). 100 g (poids humide) de produit ainsi obtenu sont ensuite introduits dans un ballon tricol muni d'un réfrigérant et d'un agitateur magnétique, 500 mL d'eau sont ajoutés ainsi qu'une enzyme protéolytique d'une activité équivalente à 3789 SAPU. La réaction est ensuite portée à 45 °C pendant 4 heures. La température est ensuite portée à 90 °C pendant 15 minutes, le mélange réactionnel est enfin filtré (0,40 - 0,45 $\mu$m). Le résidu est séché pendant 24 heures à 70 °C : il s'agit donc de la chitine obtenue par voie enzymatique de purification ; le filtrat est congelé et est lyophilisé : il s'agit donc de l'hydrolysat.

Le procédé est identique quel que soit l'insecte ou l'enzyme étudiés.

c) Analyses

Mesure du taux de cendres

Le taux de cendres a été déterminé selon la méthode relevant du règlement CE 152/2009 du 27-01-2009.

Mesure de la teneur en protéines

Le taux de protéines est obtenu par la méthode Dumas, avec le coefficient de conversion de 6,25, adaptée de la norme NF EN ISO 16634-1

Mesure de la teneur en lipides

Le taux de lipides est obtenu par une méthode adaptée du règlement CE 152/2009 - procédé B - SN.

Digestibilité pepsique

La digestibilité pepsique est mesurée par la méthode décrite dans la directive 72/199/CE.

Abondance relative en acides aminés

L'abondance des acides aminés a été déterminée par une méthode issue du règlement CE 152/2009 du 27-01-2009 - SN. La teneur en tryptophane a été déterminée séparément par une méthode adaptée du règlement CE 152/2009 du 27-01-2009 - SN. L'abondance relative a été calculée en rapportant la teneur de chaque acide aminé au taux des acides aminés.

Taux d'acides aminés

Le taux des acides aminés a été déterminé en additionnant les valeurs individuelles obtenues pour chaque acide aminé, y compris le tryptophane.

Pureté gravimétrique

La pureté gravimétrique est déterminée en comparaison de la masse en résidu sec obtenue par rapport à celle résultant d'une extraction chimique, cette dernière étant évaluée à 5% environ de la matière sèche initiale.

Pureté colorimétrique

La couleur de l'échantillon a été estimée grâce à l'analyse de photographies d'échantillons à l'aide du logiciel ImageJ selon les trois couleurs rouge, verte et bleue (RVB ou RGB en anglais), leur moyenne représentant une cotation de la couleur réelle. Un échantillon de chitine de crevettes commercialisé par Chitine France a été pris comme standard (100% de pureté) et la pureté colorimétrique des échantillons produits a été calculée en tant que pourcentage de cette couleur (ratio couleur de l'échantillon/couleur du standard).

Pureté par différence

Dans le cas de cette mesure, les quantités d'impuretés connues (acides aminés, lipides et cendres) ont été soustraites à la valeur de pureté absolue (100%) pour obtenir la valeur de la pureté estimée par différence ; *i.e.* un échantillon qui contient 30% protéines, 10% de lipides et 1% de cendres, se voit dès lors attribuer une pureté de 100-30-10-1=59%

Degré de cristallinité

Les mesures ont été effectuées selon la technique WAXS (wide angle X-ray scattering) sur l'appareil Bruker D8 Advance (A25 DaVinci design) équipé d'un détecteur Lynxeye XE. Les résultats ont été interprétés suivant le procédé décrit dans Loelovich, M. Res. Rev.: J. Chem. 2014, 3, 7-14.

**II. Hydrolysat**

a) Cendres

Le taux de cendres de l'hydrolysat issu du procédé « broyage + H2O2 + pressage » a été étudié sur divers insectes et différentes enzyme. Ces taux de cendres, indiqués en % en poids de l'hydrolysat, sont indiqués dans le Tableau 4 ci-dessous.

Tableau 4 : Taux de cendres de l'hydrolysat - différents insectes et enzymes

| | |
|---|---|
| TM + prolyve | 2,3 |
| TM + sumizyme | 2,5 |

(suite)

| | |
|---|---|
| TM + novozyme | 1,95 |
| TM + neutrase | 2,6 |
| HI + prolyve | 3,3 |
| GM + prolyve | 2,4 |
| AD + prolyve | 2,4 |

Ainsi pour l'ensemble des insectes et des enzymes testés, le taux de cendres dans l'hydrolysat est <3,5%, et même pour les insectes présentant naturellement un taux de cendres faible à modéré, ce taux est <3%.

b) Teneur en protéines

La teneur en protéines de l'hydrolysat issu du procédé « broyage + $H_2O_2$ + pressage » a été étudié sur divers insectes et différentes enzyme. Ces teneurs, indiqués en % en poids de l'hydrolysat, sont indiqués dans le Tableau 5 ci-dessous.

**Tableau 5 :** Teneur en protéines de l'hydrolysat - différents insectes et enzymes

| | |
|---|---|
| TM + prolyve | 81,2 |
| TM + sumizyme | 80 |
| TM + novozyme | 83,25 |
| TM + neutrase | 85,1 |
| HI + prolyve | 74,85 |
| GM + prolyve | 76,6 |
| AD + prolyve | 82,8 |

Pour l'ensemble des insectes et enzymes testés, le taux de protéines dans l'hydrolysat est >74%, et même pour les insectes non-volant, ce taux est ≥80%.

c) Teneur en lipides

La teneur en protéines de l'hydrolysat issu du procédé « broyage + H2O2 + pressage » a été étudié sur divers insectes et différentes enzyme. Ces teneurs, indiqués en % en poids de l'hydrolysat, sont indiqués dans le Tableau 6 ci-dessous.

**Tableau 6 :** Teneur en lipides de l'hydrolysat - différents insectes et enzymes

| | |
|---|---|
| TM + prolyve | 2,9 |
| TM + sumizyme | 4,4 |
| TM + neutrase | 2,7 |
| HI + prolyve | 8,3 |
| GM + prolyve | 9,5 |
| AD + prolyve | 3 |

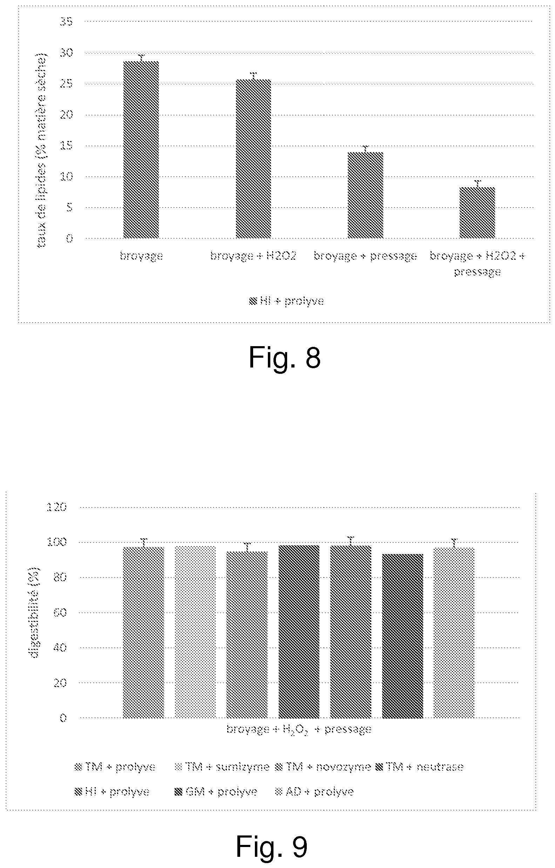

La combinaison des étapes de l'agent oxydant et de pressage contribue à significativement abaisser le taux de lipides dans l'hydrolisat (Figure 8). Ainsi ce taux est inférieur à 10% pour l'ensemble des insectes et même à 5% pour les insectes non-volants.

d) Digestibilité

Le procédé permet l'obtention d'un hydrolysat avec un taux très élevé de digestibilité, >93%, et ce quel que soit l'enzyme ou l'insecte étudié (Figure 9).

e) Abondance en acides aminés

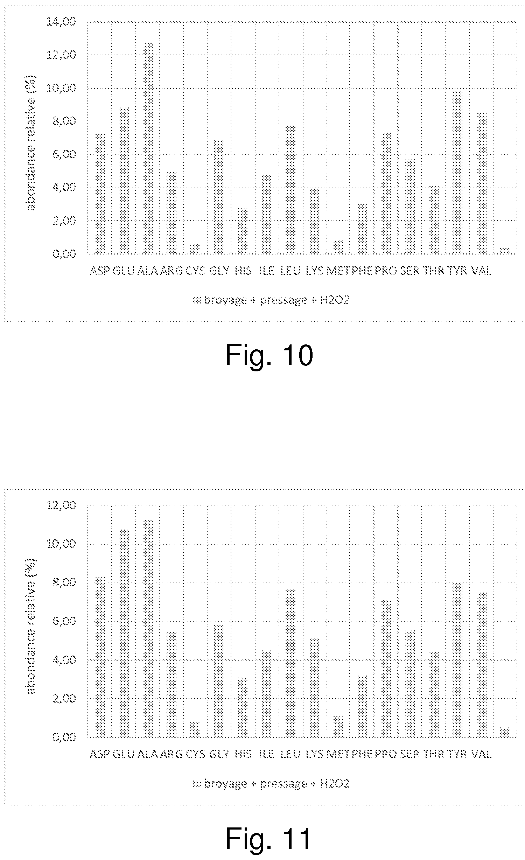

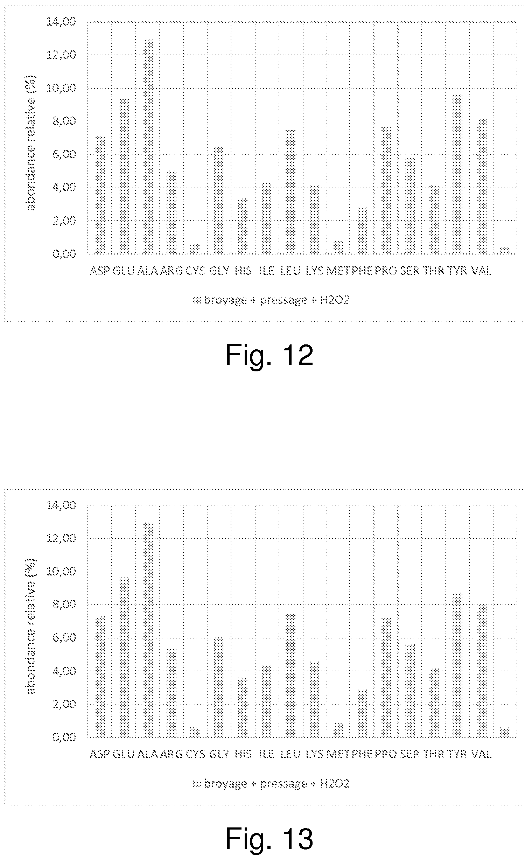

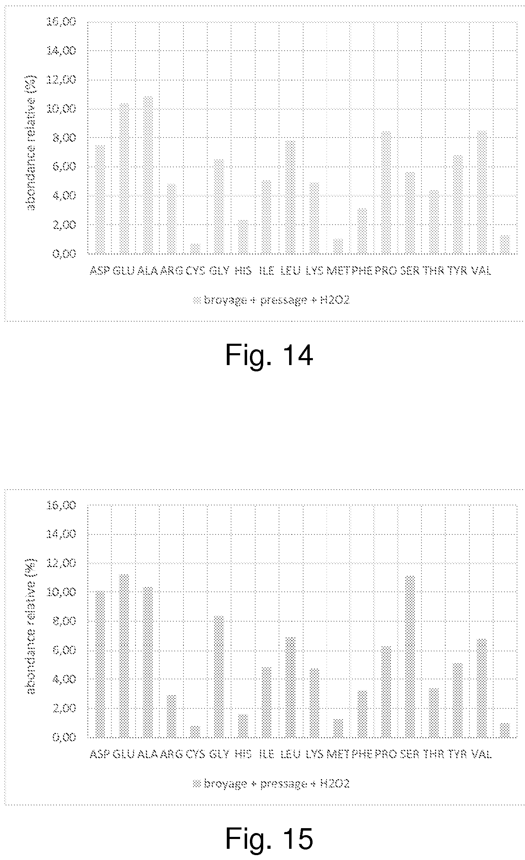

L'hydrolysat obtenu par le procédé est majoritairement composé d'acides aminés tels que l'alanine, le glutamate, l'acide aspartique et la tyrosine et, dans une moindre mesure, la valine, la sérine et la glycine, et ce quel que soit l'enzyme ou l'insecte étudiés (Figures 10 - 16).

**III. Chitine**

a) Cendres

Le taux de cendres de la chitine issue du procédé « broyage + H2O2 + pressage » a été étudié sur divers insectes et différentes enzyme. Ces taux de cendres, indiqués en % en poids de chitine, sont indiqués dans le Tableau 7 ci-dessous.

**Tableau 7 :** Taux de cendres de la chitine - différents insectes et enzymes

| | |
|---|---|
| TM + prolyve | 0,5 |
| TM + sumizyme | 0,95 |
| TM + novozyme | 0,75 |
| TM + neutrase | 0,65 |
| HI + prolyve | 2,3 |
| GM + prolyve | 0,7 |
| AD + prolyve | 2,35 |

Le taux de cendres dans la chitine obtenue par le procédé est très bas, <2,5%, quel, que soit l'enzyme ou l'insecte étudiés.

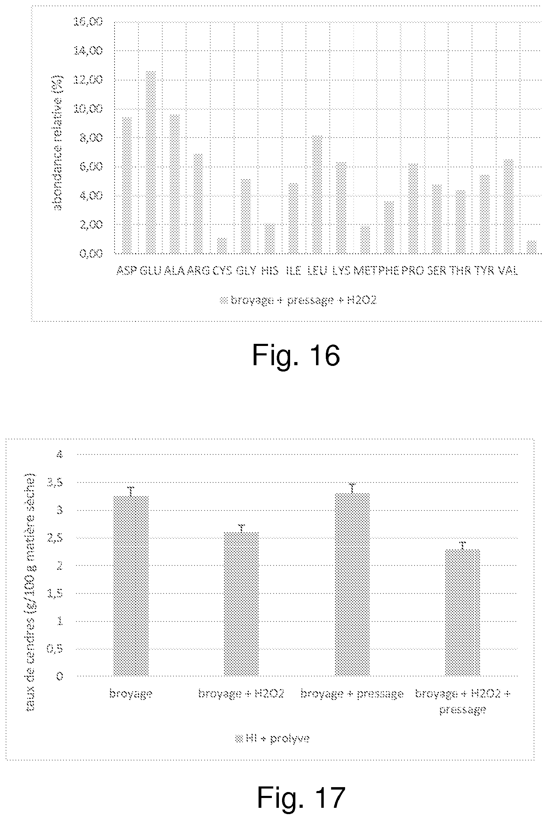

Par ailleurs, une synergie intéressante est observée lors de l'adition des étapes de pressage et de l'agent oxydant sur le taux de cendres dans la chitine obtenue (Figure 17). On arrive ainsi à obtenir avec le procédé « broyage + $H_2O_2$ + pressage » une déminéralisation pouvant atteindre 76,2% par rapport au procédé avec broyage seul, contre une déminéralisation de seulement 66,7% pour un procédé « broyage + pressage » et 63,5% pour un procédé « Broyage + H2O2 ».

b) Teneur en lipides

La teneur en lipides dans la chitine obtenue par le procédé est très faible, <6,5%, quel que soit l'insecte ou l'enzyme utilisés (Tableau 8), et même <3,2% dans le cas de *T. molitor* et ce quelle que soit l'enzyme utilisée.

**Tableau 8.** Le taux de lipides dans la chitine

| | |
|---|---|
| TM + prolyve | 3,1 |
| TM + sumizyme | 2,8 |
| TM + novozyme | 2,05 |
| TM + neutrase | 2,5 |
| GM + prolyve | 6,3 |
| AD + prolyve | 6 |

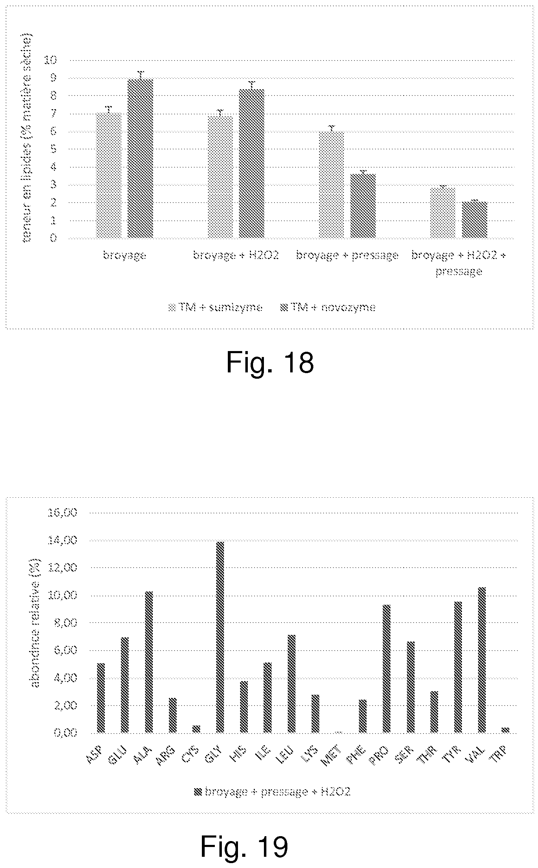

Par ailleurs, une synergie intéressante est observée lors de la combinaison des étapes d'ajout d'agent oxydant et de pressage (Figure 18). En effet, on observe dans ce cas une diminution de la teneur en lipides de 60-77%, contre 15-60% lors d'un procédé avec pressage seul et 3-3,6% lors du procédé avec ajout d'agent oxydant seul.

c) Taux et abondance d'acides aminés

Le taux d'acides aminés présents dans la chitine est relativement faible, <46%, quel que soit l'enzyme ou l'insecte étudiés (Tableau 9).

**Tableau 9.** Taux d'acides aminés présents dans la chitine

| TM + prolyve | 22,75 |
|---|---|
| TM + sumizyme | 44,74 |
| TM + novozyme | 45,45 |
| TM + neutrase | 45,20 |
| HI + prolyve | 12,97 |
| GM + prolyve | 18,92 |
| AD + prolyve | 45,56 |

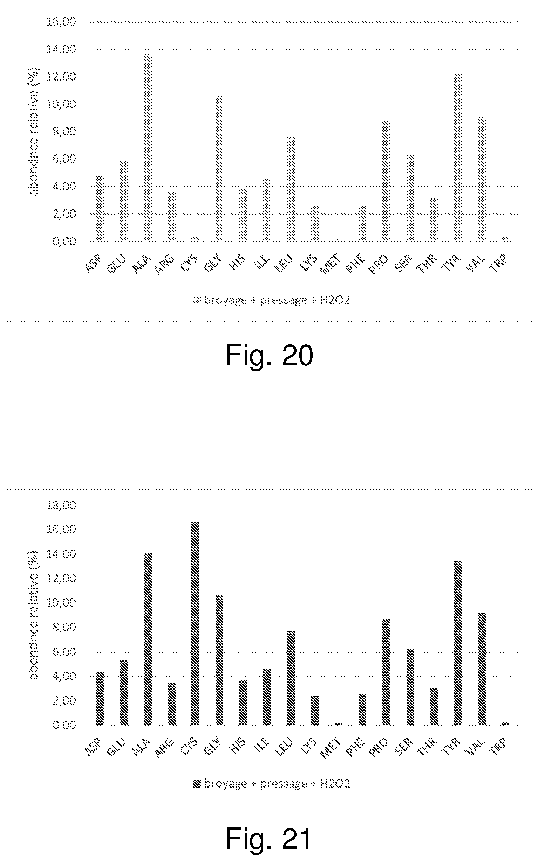

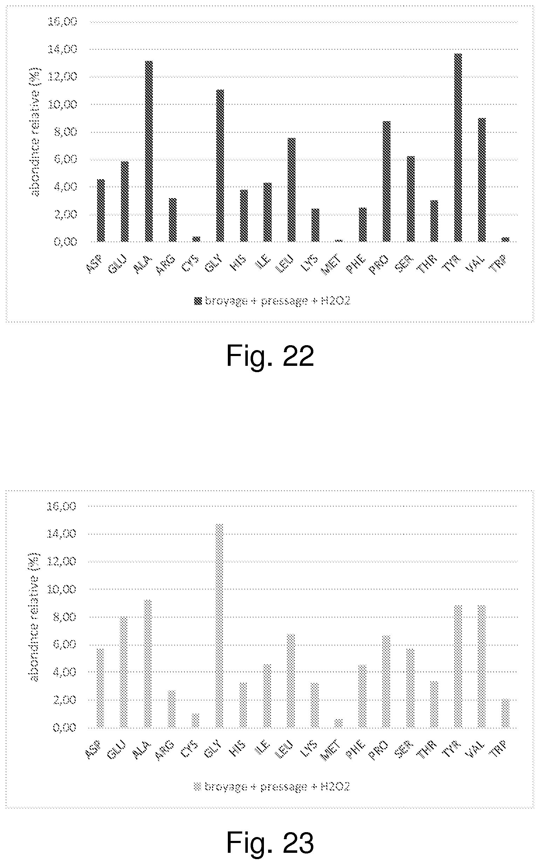

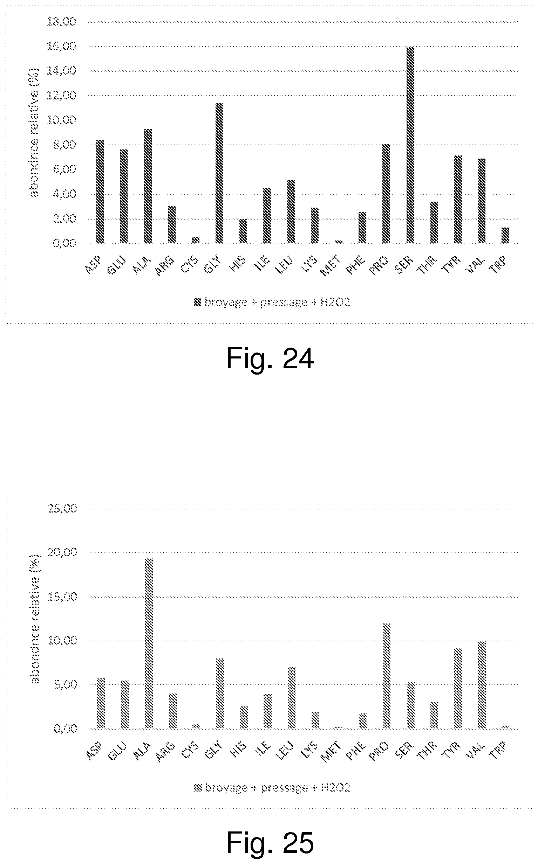

Nous pouvons constater que pour l'ensemble des insectes, l'alanine, la tyrosine et la proline, ainsi que, dans une moindre mesure, la valine, la glycine, la leucine et la sérine sont des acides aminés principalement attachés à la chitine, leur taux est compris en moyenne entre 15 - 35% de l'ensemble des acides aminés (Figures 19-25).

d) Pureté gravimétrique

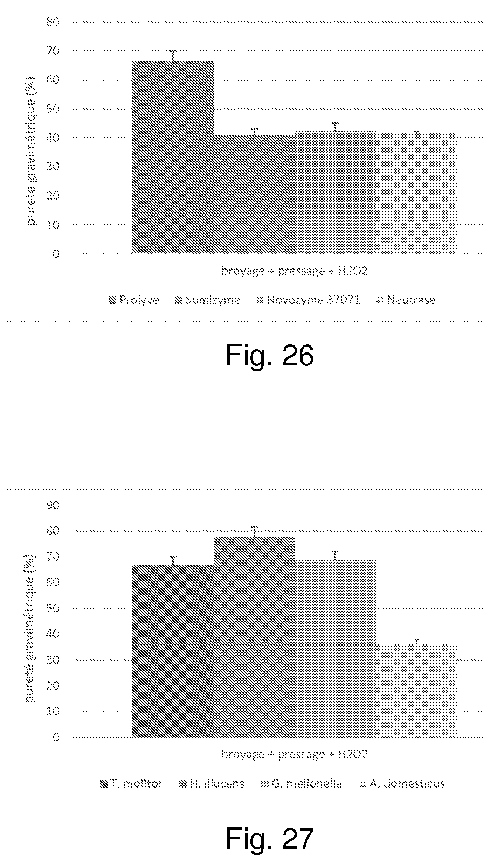

La pureté gravimétrique de la chitine ainsi obtenue est >36%, quel que soit l'insecte et même >40%, quel que soit l'enzyme dans le cas de *T. molitor* (Figures 27 et 26).

e) Pureté colorimétrique

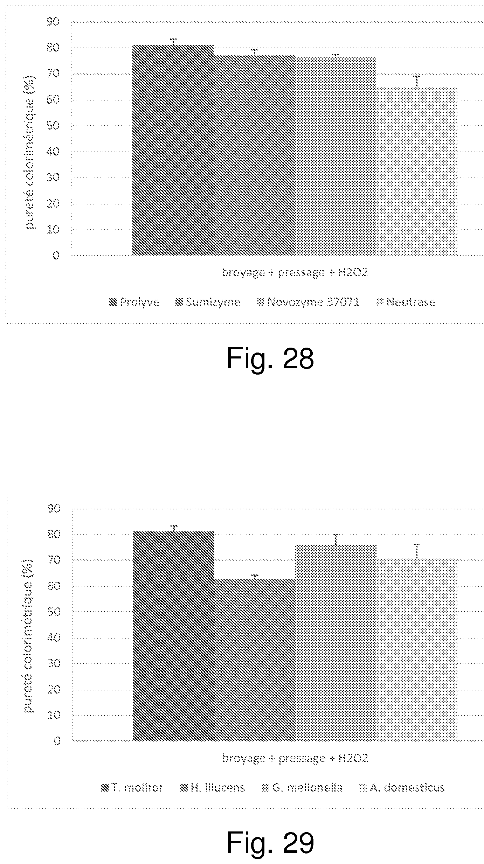

La combinaison des étapes de l'ajout d'un agent oxydant et de pressage, contribue à une pureté colorimétrique relativement élevée du produit obtenu, 62-81% quels que soient l'enzyme ou l'insecte utilisés. (Figures 28 et 29).

f) Pureté par différence

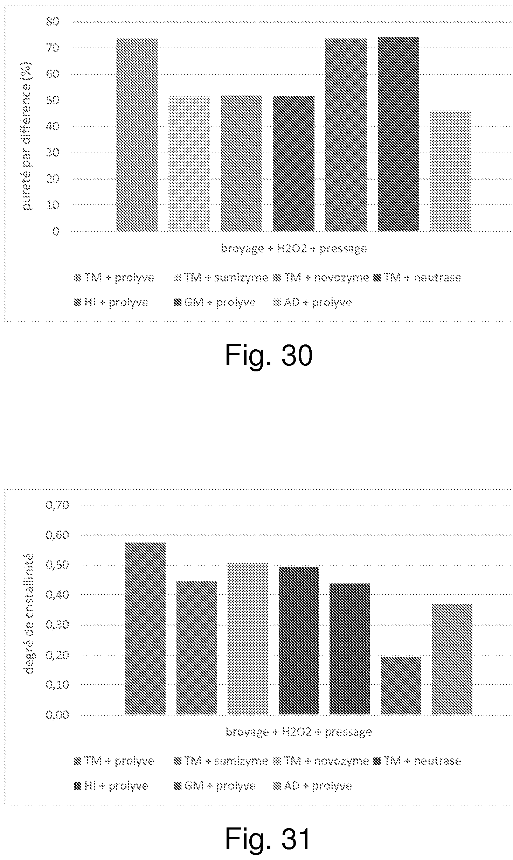

La pureté par différence des produits obtenus est relativement élevée, >46%, quels que soient les insectes et enzymes considérés et même >51% dans le cas de *T. molitor,* et pouvant aller jusqu'à 73% dans le cas *T. molitor* + prolyve (Figure 30).

g) Degré de cristallinité

Le traitement par un agent oxydant a tendance de rendre la chitine plus amorphe (Figure 31) et ce quel que soit l'insecte étudié. Le degré de cristallinité, *i.e.* le rapport des aires cristallines et amorphes, de la chitine obtenue est compris entre 0,19 et 0,58, et même dans le cas de *T. molitor* entre 0,45 et 0,58.

**Revendications**

**1.** Procédé de production de chitine et/ou de chitosan à partir de cuticules d'insectes, comportant les étapes suivantes :

(i) le pressage des cuticules d'insectes, puis,
(ii) l'hydrolyse enzymatique des cuticules d'insectes par une enzyme protéolytique,

un traitement des cuticules avec un agent oxydant ayant été effectué préalablement à l'hydrolyse enzymatique.

**2.** Procédé selon la revendication 1, comportant une étape de broyage préalable à l'étape de pressage.

**3.** Procédé selon la revendication 1 ou 2, **caractérisé en ce que** le traitement des cuticules avec un agent oxydant est effectué avant, concomitamment et/ou après l'étape de broyage et/ou de pressage.

**4.** Procédé selon l'une quelconque des revendications 1 à 3, dans lequel l'enzyme protéolytique est une protéase.

**5.** Procédé selon l'une quelconque des revendications 1 à 4, dans lequel l'agent oxydant est choisi parmi le groupe constitué par le peroxyde d'hydrogène, le permanganate de potassium, l'ozone et l'hypochlorite de sodium.

**6.** Procédé selon l'une quelconque des revendications 1 à 5, comportant une étape d'abattage des insectes préalable à l'étape de pressage et/ou de broyage.

**7.** Procédé selon la revendication 6, **caractérisé en ce que** l'étape d'abattage des insectes est une étape d'ébouillantage.

**8.** Procédé selon la revendication 6 ou 7, **caractérisé en ce que** le traitement des cuticules avec un agent oxydant est effectué concomitamment et/ou après l'étape d'abattage.

**9.** Procédé selon l'une quelconque des revendications 1 à 8, dans lequel le ou les insecte(s) est/sont choisi(s) parmi le groupe constitué par les coléoptères, les lépidoptères, les orthoptères et les diptères.

**10.** Procédé de production de chitine à partir d'insectes, comportant les étapes suivantes :

a) l'abattage des insectes,
b) le broyage des insectes,
c) le pressage des insectes,
d) l'hydrolyse enzymatique des cuticules d'insectes par une enzyme protéolytique,
e) la récupération de la chitine,

les cuticules d'insectes étant traitées avec un agent oxydant, avant l'étape d).

**11.** Procédé de production de chitosan à partir d'insectes, comportant les étapes suivantes :

a) l'abattage des insectes,
b) le broyage des insectes,
c) le pressage des insectes,
d) l'hydrolyse enzymatique des cuticules d'insectes par une enzyme protéolytique,
e) la récupération de la chitine,
f) la désacétylation de la chitine récupérée,
g) la récupération du chitosan,

les cuticules d'insectes étant traitées avec un agent oxydant, avant l'étape d).

**Patentansprüche**

**1.** Verfahren zur Herstellung von Chitin und/oder Chitosan ausgehend von Insekten-Cuticulae, welches die folgenden Schritte umfasst:

(i) Pressen der Insekten-Cuticulae, dann
(ii) enzymatische Hydrolyse der Insekten-Cuticulae durch ein proteolytisches Enzym,

wobei vor der enzymatischen Hydrolyse eine Behandlung der Cuticulae mit einem Oxidationsmittel durchgeführt worden ist.

**2.** Verfahren nach Anspruch 1, welches einen dem Pressschritt vorangehenden Zerkleinerungsschritt umfasst.

**3.** Verfahren nach Anspruch 1 oder 2, **dadurch gekennzeichnet, dass** die Behandlung der Cuticulae mit einem Oxidationsmittel vor, gleichzeitig mit und/oder nach dem Zerkleinerungs- und/oder Pressschritt durchgeführt wird.

**4.** Verfahren nach einem der Ansprüche 1 bis 3, in welchem das proteolytische Enzym eine Protease ist.

**5.** Verfahren nach einem der Ansprüche 1 bis 4, in welchem das Oxidationsmittel aus der Gruppe ausgewählt ist, die aus Wasserstoffperoxid, Kaliumpermanganat, Ozon und Natriumhypochlorit besteht.

**6.** Verfahren nach einem der Ansprüche 1 bis 5, welches einen dem Press- und/oder Zerkleinerungsschritt vorangehenden Schritt der Tötung der Insekten umfasst.

**7.** Verfahren nach Anspruch 6, **dadurch gekennzeichnet, dass** der Schritt der Tötung der Insekten ein Abbrühschritt ist.

**8.** Verfahren nach Anspruch 6 oder 7, **dadurch gekennzeichnet, dass** die Behandlung der Cuticulae mit einem Oxidationsmittel gleichzeitig mit und/oder nach dem Tötungsschritt durchgeführt wird.

**9.** Verfahren nach einem der Ansprüche 1 bis 8, in welchem das Insekt oder die Insekten aus der Gruppe ausgewählt ist/sind, die aus den Coleoptera, den Lepidoptera, den Orthoptera und den Diptera besteht.

**10.** Verfahren zur Herstellung von Chitin ausgehend von Insekten, welches die folgenden Schritte umfasst:

    a) Töten der Insekten,
    b) Zerkleinern der Insekten,
    c) Pressen der Insekten,
    d) enzymatische Hydrolyse der Insekten-Cuticulae durch ein proteolytisches Enzym,
    e) Gewinnen des Chitins,

wobei die Insekten-Cuticulae vor dem Schritt d) mit einem Oxidationsmittel behandelt werden.

**11.** Verfahren zur Herstellung von Chitosan ausgehend von Insekten, welches die folgenden Schritte umfasst:

    a) Töten der Insekten,
    b) Zerkleinern der Insekten,
    c) Pressen der Insekten,
    d) enzymatische Hydrolyse der Insekten-Cuticulae durch ein proteolytisches Enzym,
    e) Gewinnen des Chitins,
    f) Desacetylieren des gewonnenen Chitins,
    g) Gewinnen des Chitosans,

wobei die Insekten-Cuticulae vor dem Schritt d) mit einem Oxidationsmittel behandelt werden.

**Claims**

**1.** A method for producing chitin and/or chitosan from insect cuticles, comprising the following steps:

    (i) pressing of the insect cuticles, then,
    (ii) enzymatic hydrolysis of the insect cuticles by a proteolytic enzyme,

the cuticles having been treated with an oxidising agent prior to enzymatic hydrolysis.

**2.** A method according to claim 1, comprising a grinding step prior to the pressing step.

**3.** A method according to claim 1 or claim 2, **characterised in that** the cuticles are treated with an oxidising agent before, concomitantly with and/or after the grinding and/or pressing steps.

**4.** A method according to any one of claims 1 to 3, in which the proteolytic enzyme is a protease.

**5.** A method according to any one of claims 1 to 4, in which the oxidising agent is selected from among the group constituted by hydrogen peroxide, potassium permanganate, ozone and sodium hypochlorite.

6. A method according to any one of claims 1 to 5, comprising an insect killing step prior to the pressing and/or grinding steps.

7. A method according to claim 6, **characterised in that** the insect killing step is a scalding step.

8. A method according to claim 6 or claim 7, **characterised in that** the cuticles are treated with an oxidising agent concomitantly with and/or after the killing step.

9. A method according to any one of claims 1 to 8, in which the insect(s) is/are selected from the group constituted by coleoptera, lepidoptera, orthoptera and diptera.

10. A method for producing chitin from insects, comprising the following steps:

   a) killing of the insects,
   b) grinding of the insects,
   c) pressing of the insects,
   d) enzymatic hydrolysis of the insect cuticles by a proteolytic enzyme,
   e) recovery of the chitin,

   the insect cuticles being treated with an oxidising agent before step d).

11. A method for producing chitin from insects, comprising the following steps:

   a) killing of the insects,
   b) grinding of the insects,
   c) pressing of the insects,
   d) enzymatic hydrolysis of the insect cuticles by a proteolytic enzyme,
   e) recovery of the chitin,
   f) deacetylation of the recovered chitin,
   g) recovery of the chitosan,

   the insect cuticles being treated with an oxidising agent before step d).

Fig. 1

EP 3 240 904 B1

Fig. 2

□ chitine par hydrolyse enzymatique

⊞ chitine par hydrolyse enzymatique + H2O2 pendant l'ébouillantage

Ⅲ chitine par hydrolyse enzymatique + H2O2 après hydrolyse

■ chitine par voie chimique

Fig. 3

Fig. 4

taux des lipides (%) dans l'intermédiaire dont la chitine a été extraite

▨ pureté de la chitine
● tx des lipides dans l'hydrolysat
⬚ tx des lipides dans la chitine

Fig. 5

Fig. 6

tx de lipides dans le gâteau de presse
tx de lipides dans le jus de presse

Fig. 7

Fig. 8

Fig. 9

Fig. 10

Fig. 11

Fig. 12

Fig. 13

Fig. 14

Fig. 15

Fig. 16

Fig. 17

Fig. 18

Fig. 19

Fig. 20

Fig. 21

Fig. 22

Fig. 23

Fig. 24

Fig. 25

Fig. 26

Fig. 27

Fig. 28

Fig. 29

Fig. 30

Fig. 31

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **LOELOVICH.** *M. Res. Rev.: J. Chem.,* 2014, vol. 3, 7-14